# EUROPEAN PATENT APPLICATION

(11) **EP 1 721 981 A1**
(43) Date of publication of application: **15.11.2006**
(21) Application number: 05719956.4
(22) Date of filing: 03.03.2005
(51) Int. Cl.: C12N 15/38, A61K 39/25, A61P 31/22, C12N 5/10, C12N 7/00

(54) **RECOMBINANT VARICELLA-ZOSTER VIRUS**

(30) Priority: 05.03.2004 JP 2004063277
(71) Applicant: THE RESEARCH FOUNDATION FOR MICROBIAL DISEASES OF OSAKA UNIVERSITY, Suita-shi, Osaka 565-0871 (JP)
(72) Inventor: NAGAIKE, Kazuhiro, Mitoyo-gun, Kagawa 7680100 (JP); MORI, Yasuko, Ibaraki-shi, Osaka 5670046 (JP); GOMI, Yasuyuki, Kanonji-shi, Kagawa 7680001 (JP); TAKAHASHI, Michiaki, Suita-shi, Osaka 5650875 (JP); YAMANISHI, Kouichi, Toyonou-cho, Osaka 5630104 (JP)
(74) Representative: Bates, Philip Ian
(86) International application number: PCT/JP2005/003652
(87) International publication number: WO 2005/085445

(57) **Abstract**

A recombinant varicella-zoster virus; a process for producing the same; a pharmacological composition containing recombinant varicella-zoster virus; a vector containing a genomic gene of varicella-zoster virus and BAC vector sequence; cells containing the above vector; a fragment capable of homologous recombination with a genome of varicella-zoster virus; and a nucleic acid cassette containing the BAC vector sequence. For these, there is provided a process for producing recombinant varicella-zoster virus, comprising use of the BAC vector sequence.

## Description

### 1. FIELD OF THE INVENTION:

The present invention relates to recombinant varicella-zoster virus, particularly recombinant varicella-zoster virus prepared using BAC (*E. coli* artificial chromosome), and a pharmaceutical composition comprising such a virus. Further, the present invention relates to a vector comprising a varicella-zoster virus genomic gene and a BAC vector sequence, and a cell containing such a vector. Further, the present invention relates to a method for producing recombinant varicella-zoster virus. Further, the present invention relates to a nucleic acid cassette comprising a fragment capable of homologous recombination with a varicella-zoster virus genome, and a BAC vector sequence.

### 2. DESCRIPTION OF THE RELATED ART:

Varicella-zoster virus (VZV) is a virus whichbelongs to viruses of the family *Herpesviridae,* and is responsible for diseases (varicella and zoster) which exhibit two different presentations. Early infection to this virus causes varicella (chicken pox). Then, the virus latently infects the ganglion. After a long period of time, this virus is reactivated by some cause, and then presents as zoster, which is a symptom that presents when virus particles are formed, the virus particles arrive at the epidermic cell through a nerve celland form varicella in the region where nerve cells are present.

The VZV genome is double-stranded DNA of about 125000 bases. The whole base sequence has been determined by Davison et *al.* It is known that at least 72 genes are present on the genome.

The development of VZV vaccine is difficult. Oka strain of VZV vaccine is the one and only vaccine for a varicella virus in the world, developed by Takahashi, et al. (Japanese Laid-Open Publication No. 53-41202). The existing attenuated live varicella vaccine has been produced by employing virus derived from the attenuated live varicella virus Oka strain used as a seed, and has been practiced all over the world (Requirements for Varicella Vaccine (Live) Adopted 1984; Revised 1993: WHO Technical Report Series, No. 848, pp.22-38, 1994). This Oka strain is obtained from a virus (Oka original strain) isolated from an affected infant that presents typical varicella, by passage through several generations employing human diploid cell after passage through 12 generations, employing human embryonic lung cell at 34 centigrade, and through 11 generations by employing guinea-pig embryonal cell. The Oka original strain is of high pathogenicity. On the contrary, it is recognized that the Oka vaccine strain (Oka strain) has very little side-effects to a normal child. As such, the Oka strain is useful as a vaccine strain having very little pathogenicity.

A virus vaccine has a possibility of changing its genotype of the virus through passage. There is also a possibility that the Oka strain has a genetic variety because a lot of passages are done in the process of preparing the Oka strain. Practically, to ensure its safety and effectiveness, considering genetic changes of a virus through passages in the process of producing a vaccine, a seed lot systemhas been established that limits the number of passages of a varicella seed virus which is approved to be produced, that is, employing a virus as a vaccine within 10 generations from the total number of passages, on the basis that the number of passages is 0 at the time of approval of seed.

On the other hand, from the follow-up of the effect of a varicella vaccine and post-marketing Surveillance (PMS), or in terms of epidemiology, the analysis of virological difference between a fresh field strain of varicella virus which is separated from a varicella patient by natural infection and a vaccine strain derived from the above-mentioned Oka strain has been necessary, resulting in that the various analyses employing the techniques from immunology, genetic engineering, and the like have been already done. For example, the judgment based on trials, such as the differences of gene structures, DNAbase sequences, and the like between varicella virus strains (Journal of General Virology, 59, 660-668, 1986; 67, 1759-1816, 1986), presence of restriction enzyme Pst I site (Japanese Journal of Experimental Medicine, 59, 233-237, 1989), RFLP (Restriction Fragment Length Polymorphism) employing PCR (Polymerase Chain Reaction) (Journal of Virology, 66, 1016-1020, 1992), and the combination of the above-mentioned presence of Pst I site and RFLP (Journal of Clinical Microbiology, 33, 658-660, 1995), have been reported. Although these trials propose conditions for identifying a fresh field strain from the vaccine strain derived from the Oka strain, it lacks the reliability and is not conclusive because of the genetic variety of the Oka Strain itself, and therefore, there still exist problems in terms of quality control. Further, the method of identifying Oka strain of a varicella virus by employing the gene 14 region of a varicella virus (US Patent No. 6, 093, 535), the method of identifying a virus strain for an attenuated live varicella vaccine by employing the gene 62 region (International Publication No. WO 00/50603), and the like are also known. These techniques make it possible to identify the differences among the following three strains; the Oka strain of varicella virus (high virulent parent strain), a vaccine strain derived therefrom (an attenuated Oka strain), and a varicella virus strain other than the Oka strain. However, the criterion of preparation of an attenuated live varicella vaccine for quality control and quality assurance would not be sufficient.

As it is now, quality control by evaluating and identifying the quality of vaccine, such as by direct or quantitative gene analysis for genome DNA of seed virus and vaccine virus has not been practiced. Therefore, the accuracy of quality control and quality assurance of an attenuated strain for live vaccine cannot be calculated, and therefore, is ambiguous. Accordingly, to increase the accuracy of quality control and quality assurance is extremely important to secure and ensure the effectiveness, safety, and homogeneity of an attenuated live varicella vaccine. However, as mentioned above, the method for the foregoing has not been established, and the problems have still remained to be solved as tasks of pressing urgency.

Also, to develop an altered varicella-zoster virus vaccine which is superior to the Oka strain, recombinant varicella-zoster virus by mutagenesis, and a production method thereof has been desired.

### DISCLOSURE OF THE INVENTION

### THE PROBLEM TO BE SOLVED BY THE INVENTION

An object of the present invention is to increase the accuracy of quality control and quality assurance, and securing and ensuring the effectiveness, safety, and homogeneity of an attenuated live varicella vaccine. Further, a problem to be solved by the present invention is, in order to develop variant varicella-zoster virus vaccine superior to Oka strain, establishing a method to produce recombinant varicella-zoster virus by mutagenesis, and producing such a virus.

To achieve this, the present invention provides recombinant varicella-zoster virus, and a production method thereof, e.g., a method for producing recombinant varicella-zoster virus from a single virus strain using a BAC (Bacterial artificial chromosome).

### SUMMARY OF THE INVENTION

The present inventors developed a method for producing recombinant varicella-zoster virus using a BAC vector sequence to complete the present invention.

Therefore, the present invention provides the following.
1. A recombinant varicella-zoster virus.
2. A recombinant varicella-zoster virus of item 1, comprising a BAC vector sequence.
3. The recombinant varicella-zoster virus of item 2, wherein at least part of the BAC vector sequence is inserted into a non-essential region of a varicella-zoster virus genome.
4. The recombinant varicella-zoster virus of item 3, wherein the non-essential region is selected from the group consisting of the following regions:
   the region in the ORF of gene 7, the region in the ORF of gene 8, the region in the ORF of gene 9, the region in the ORF of gene 10, the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 62, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.
5. The recombinant varicella-zoster virus of item 4, wherein the non-essential region is the region flanking the ORF of gene 11, or the region flanking the ORF of gene 12.
6. The recombinant varicella-zoster virus of item 2, wherein at least part of the BAC vector sequence is inserted into the region in the ORF of gene 62 of a varicella-zoster virus genome.
7. The recombinant varicella-zoster virus of item 2, wherein the BAC vector sequence comprises a recombinant protein dependent recombinant sequence.
8. The recombinant varicella-zoster virus of item 2, wherein the BAC vector sequence comprises a selectable marker.
9. The recombinant varicella-zoster virus of item 8, wherein the selectable marker is a drug selectable marker.
10. The recombinant varicella-zoster virus of item 2, wherein the selectable marker is a gene encoding green fluorescent protein.
11. The recombinant varicella-zoster virus of item 2, wherein the varicella-zoster virus genome is derived from a wild type strain.
12. The recombinant varicella-zoster virus of item 2, wherein the varicella-zoster virus genome is derived from a mutant type strain.
13. The recombinant varicella-zoster virus of item 2, wherein the varicella-zoster virus genome is derived from Oka vaccine strain.
14. The recombinant varicella-zoster virus of item 2, wherein the varicella-zoster virus genome has mutations in gene 62 and gene 6.
15. The recombinant varicella-zoster virus of item 14, wherein the gene 62 comprises at least the base substitutions of the following (a)-(d) in SEQ ID NO. 5:
   (a) base substitution at position 2110 for G;
   (b) base substitution at position 3100 for G;
   (c) base substitution at position 3818 for C; and
   (d) base substitution at position 4006 for G,
      and, the gene 6 comprises at least a base substitution at porision 5745 for G, in SEQ ID NO. 8.
16. The recombinant varicella-zoster virus of item 2, wherein the BAC vector sequence comprises the sequence set forth in SEQ ID NO.: 7.
17. A pharmaceutical composition comprising the virus of item 1.
18. The pharmaceutical composition of item 17, wherein the composition is in the form of a vaccine.
19. A vector comprising a varicella-zoster virus essential gene and a BAC vector sequence other than the gene 62.
20. The vector of item 19, further comprising the gene 62.
21. The vector of item 19, wherein a mammalian cell produces a varicella-zoster virus when the vector is introduced into the mammalian cell.
22. The vector of item 19, wherein a portion where a sequence derived from the varicella-zoster virus genome is linked to the BAC vector sequence is within a non-essential region of the varicella-zoster virus genome.
23. The vector of item 22, wherein the non-essential region is selected from the group consisting of the following regions of:
   the region in the ORF of gene 7, the region in the ORF of gene 8, the region in the ORF of gene 9, the region in the ORF of gene 10, the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 62, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.
24. The vector of item 23, wherein the portion for linking is the region flanking the ORF of gene 11 and the region flanking the ORF of gene 12.
25. The vector of item 19, wherein a portion where a sequence derived from the varicella-zoster virus genome is linked to the BAC vector sequence is in the ORF of gene 62 of the varicella-zoster virus genome.
26. The vector of item 19, wherein the BAC vector sequence comprises recombinant protein dependent recombinant sequence.
27. The vector of item 19, wherein the BAC vector sequence comprises a selectable marker.
28. The vector of item 27, wherein the selectable marker is drug selectable marker.
29. The vector of item 27, wherein the selectable marker is a gene encoding green fluorescent protein.
30. The vector of item 19, wherein the varicella-zoster virus genome is derived from a wild type strain.
31. The vector of item 19, wherein the varicella-zoster virus genome is derived from a mutant type strain.
32. The vector of item 19, wherein the varicella-zoster virus genome is derived from Oka vaccine strain.
33. The vector of item 19, wherein the varicella-zoster virus genome has mutations in gene 62 and gene 6.
34. The vector of item 33, wherein the gene 62 comprises at least the base substitutions of the following (a)-(d) in SEQ ID NO. 5:
   (a) base substitution at position 2110 for G;
   (b) base substitution at position 3100 for G;
   (c) base substitution at position 3818 for C; and
   (d) base substitution at position 4006 for G,
      and, the gene 6 comprises at least a base substitution at position 5745 for G, in SEQ ID NO. 8.
35. The vector of item 19, wherein the BAC vector sequence comprises the sequence set forth in SEQ ID NO.: 7.
36. A cell comprising the vector of item 19.
37. The cell of item 36, wherein the cell is a bacterial cell.
38. The bacterial cell of item 37, wherein the bacterial cell is E. *coli.*
39. The cell of item 36, wherein the cell is a mammalian cell.
40. The mammalian cell of item 39, wherein the mammalian cell is derived from human.
41. A virus produced by the mammalian cell of item 39.
42. A pharmaceutical composition comprising the virus of item 41.
43. The pharmaceutical composition of item 42, wherein the composition is in the form of a vaccine.
44. A method to produce recombinant varicella-zoster virus, comprising:
   introducing a vector comprising a varicella-zoster virus genome essential gene other than gene 62 and a BAC vector sequence into a mammalian host cell; and
   culturing the mammalian host cell to produce recombinant varicella-zoster virus.
45. The method of item 44, wherein the vector further comprises the gene 62.
46. The method of item 44, wherein the mammalian host cell is derived from human.
47. The method of item 44, wherein the BAC vector sequence comprises at least two recombinant protein dependent recombinant sequences.
48. The method of item 47, further comprising a step of recombination between the two recombinant protein dependent recombinant sequences.
49. The method of item 44, wherein a portion where a sequence derived from the varicella-zoster virus genome is linked to the BAC vector sequence is within a non-essential region of the varicella-zoster virus genome.
50. The method of item 49, wherein the non-essential region is selected from the group consisting of the following regions of:
   the region in the ORF of gene 7, the region in the ORF of gene 8, the region in the ORF of gene 9, the region in the ORF of gene 10, the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 62, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.
51. The vector of item 50, wherein the portion which is linked is the region flanking the ORF of gene 11 or the region flanking the ORF of gene 12.
52. The vector of item 44, wherein a portion where a sequence derived from the varicella-zoster virus genome is linked to the BAC vector sequence is in the ORF of gene 62 of the varicella-zoster virus genome.
53. The method of item 44, wherein the BAC vector sequence comprises a recombinant protein dependent recombinant sequence.
54. The method of item 44, wherein the BAC vector sequence comprises a selectable marker.
55. The method of item 54, wherein the selectable marker is a drug selectable marker.
56. The method of item 54, wherein the selectable marker is a gene encoding green fluorescent protein.
57. The method of item 44, wherein the varicella-zoster virus genome is derived from a wild type strain.
58. The method of item 44, wherein the varicella-zoster virus genome is derived from a mutant type strain.
59. The method of item 44, wherein the varicella-zoster virus genome is derived from Oka vaccine strain.
60. The method of item 44, wherein the varicella-zoster virus genome has mutations in gene 62 and gene 6.
61. The method of item 60, wherein the gene 62 comprises at least the base substitutions of the following (a)-(d) in SEQ ID NO. 5:
   (a) base substitution at position 2110 for G;
   (b) base substitution at position 3100 for G;
   (c) base substitution at position 3818 for C; and
   (d) base substitution at position 4006 for G,
      and, the gene 6 comprises at least a base substitution at position 5745 for G, in SEQ ID NO. 8.
62. The method of item 44, wherein the BAC vector sequence comprises the sequence set forth in SEQ ID NO.: 7.
63. A virus produced by the method of item 44.
64. A pharmaceutical composition comprising the virus of item 63.
65. The pharmaceutical composition of item 64, wherein the composition is in the form of a vaccine.
66. A method to introduce a mutation into the vector of item 19, comprising:
   introducing the vector into a bacterial host cell;
   introducing a plasmid vector comprising a fragment consisting of a portion of varicella-zoster virus genome into the bacterial host cell, wherein the fragment has at least one mutation;
   culturing the bacterial host cell;
   isolating a vector having BAC sequence from the cultured bacterial host cell.
67. A method to introduce a mutation into the vector of item 19, comprising:
   introducing the vector into a bacterial host cell;
   introducing a first plasmid vector comprising a first fragment consisting of a portion of varicella-zoster virus genome into the bacterial host cell, wherein the first fragment has at least one mutation;
   introducing a second plasmid vector comprising a second fragment consisting of a portion of varicella-zoster virus genome into the bacterial host cell, wherein the second fragment has at least one mutation, and the first fragment is different from the second fragment;
   culturing the bacterial host cell;
   isolating a vector having BAC sequence from the cultured bacterial host cell.
68. A nucleic acid cassette comprising a first fragment which can recombine with varicella-zoster virus genome in a bacterial cell, BAC vector sequence, and a second fragment which can recombine with varicella-zoster virus genome in a bacterial cell,
   wherein the both ends of the BAC sequence are linked to the first and the second fragment, respectively.
69. The nucleic acid cassette of item 68, wherein the first fragment and the second fragment are at least 1 kb.
70. The nucleic acid cassette of item 68, wherein the first fragment and the second fragment are at least 1.5 kb.
71. The nucleic acid cassette of item 68, wherein the first fragment and the second fragment are at least 2 kb.
72. The nucleic acid cassette of item 68, wherein the first fragment and the second fragment are at least 80% identical with a varicella-zoster virus genome sequence.
73. The nucleic acid cassette of item 68, wherein the first fragment and the second fragment are at least 85% identical with a varicella-zoster virus genome sequence.
74. The nucleic acid cassette of item 68, wherein the first fragment and the second fragment are at least 90% identical with a varicella-zoster virus genome sequence.
75. The nucleic acid cassette of item 68, wherein the first fragment and the second fragment are at least 95% identical with a varicella-zoster virus genome sequence.
76. The nucleic acid cassette of item 68, wherein each of the first fragment and the second fragment are independently selected from the group consisting of the following regions of varicella-zoster virus genome:
   the region in the ORF of gene 7, the region in the ORF of gene 8, the region in the ORF of gene 9, the region in the ORF of gene 10, the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 62, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.
77. The nucleic acid cassette of item 68, wherein each of the first fragment and the second fragment is independently at least 80% identical with the region selected from the group consisting of the following regions of varicella-zoster virus genome:
   the region in the ORF of gene 7, the region in the ORF of gene 8, the region in the ORF of gene 9, the region in the ORF of gene 10, the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 62, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.
78. The nucleic acid cassette of item 68, wherein each of the first fragment and the second fragment is independently at least 85% identical with the region selected from the group consisting of the following regions of varicella-zoster virus genome:
   the region in the ORF of gene 7, the region in the ORF of gene 8, the region in the ORF of gene 9, the region in the ORF of gene 10, the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 62, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.
79. The nucleic acid cassette of item 68, wherein each of the first fragment and the second fragment is independently at least 90% identical with the region selected from the group consisting of the following regions of varicella-zoster virus genome:
   the region in the ORF of gene 7, the region in the ORF of gene 8, the region in the ORF of gene 9, the region in the ORF of gene 10, the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 62, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.
80. The nucleic acid cassette of item 68, wherein each of the first fragment and the second fragment is independently at least 95% identical with the region selected from the group consisting of the following regions of varicella-zoster virus genome:
   the region in the ORF of gene 7, the region in the ORF of gene 8, the region in the ORF of gene 9, the region in the ORF of gene 10, the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 62, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.
81. The nucleic acid cassette of item 68, wherein the first fragment and the second fragment are derived from different regions.
82. The nucleic acid cassette of item 72, wherein each of the first fragment and the second fragment are independently from the region flanking the ORF of gene 11 and the region flanking the ORF of gene 12.
83. The nucleic acid cassette of item 68, wherein the BAC vector sequence comprises a recombinant protein dependent recombinant sequence.
84. The nucleic acid cassette of item 68, wherein the BAC vector sequence comprises a selectable marker.
85. The nucleic acid cassette of item 84, wherein the selectable marker is drug selectable marker.
86. The nucleic acid cassette of item 68, wherein the selectable marker is a gene encoding green fluorescent protein.
87. The nucleic acid cassette of item 68, wherein the varicella-zoster virus genome is derived from a wild type strain.
88. The nucleic acid cassette of item 68, wherein the varicella-zoster virus genome is derived from a mutant type strain.
89. The nucleic acid cassette of item 68, wherein the varicella-zoster virus genome is derived from Oka vaccine strain.
90. The nucleic acid cassette of item 68, wherein the BAC vector sequence comprises the sequence set forth in SEQ ID NO.: 7.
91. The nucleic acid cassette of item 68, having a nucleic acid sequence set forth in SEQ ID NO.: 2.

The present invention provides recombinant varicella-zoster virus, and a production method thereof. For example, the present invention provides a method for producing recombinant varicella-zoster virus from a single viral strain using a BAC (*E. coli* artificial chromosome), and recombinant varicella-zoster virus produced by the method. Further, the present invention provides a pharmaceutical composition comprising recombinant varicella-zoster virus.

Further, the present invention provides a vector comprising a varicella-zoster viral genomic gene and a BAC vector sequence, and a cell containing such a vector, and a nucleic acid cassette comprising a fragment capable of homologous recombination with a varicella-zoster virus genome, and a BAC vector sequence.

### BRIEF DESCRIPTION OF THE DRAWINGS

Figure 1 is a schematic diagram showing the production of the Oka strain genome of varicella-zoster virus and recombinant varicella-zoster virus.
Figure 2 shows the comparison of proliferation in vitro between the Oka strain genome of varicella-zoster virus (parent strain) and the recombinant varicella-zoster virus (rV02).

### DESCRIPTION OF THE PREFERRED EMBODIMENTS

Hereinafter, the present invention will be described. It should be understood throughout the present specification that expression of a singular form includes the concept of their plurality unless otherwise mentioned. It should be also understood that the terms as used herein have definitions typically used in the art unless otherwise mentioned. Thus, unless otherwise defined, all scientific and technical terms have the same meanings as those generally used by those skilled in the art to which the present invention pertains. If there is contradiction, the present specification (including the definition) precedes.

Hereinafter, the present invention will be described. It should be understood throughout the present specification that expression of a singular form includes the concept of their plurality unless otherwise mentioned. Accordingly, for example, it should be understood that a singular article (for example, "a", "an", "the" in English) comprises the concepts of plural form unless otherwise mentioned. It should be also understood that the terms as used herein have definitions typically used in the art unless otherwise mentioned. Thus, unless otherwise defined, all scientific and technical terms have the same meanings as those generally used by those skilled in the art to which the present invention pertains. If there is contradiction, the present specification (including the definition) precedes.

### (Definition of Terms)

The definitions of terms used herein are described below.

As used herein, the term "essential gene" in relation to varicella-zoster virus refers to a gene which is essential for the growth of the varicella-zoster virus. Also, the term "non-essential gene" in relation to varicella-zoster virus refers to a gene which is not essential for the growth of the varicella-zoster virus, and in the absence of which the varicella-zoster virus can grow. Examples of non-essential genes of human varicella-zoster virus include, but are not limited to: gene 7, gene 8, gene 9, gene 10, gene 11, gene 12, gene 13, gene 14, gene 15, gene 17, gene 18, gene 19, gene 38, gene 39, gene 46, gene 47, gene 48, gene 49, gene 50, gene 56, gene 57, gene 58, gene 59, gene 61, gene 63, gene 64, gene 65, gene 66, gene 67, gene 68, gene 69, gene 70.

When a gene in a viral genome is an essential gene, the virus cannot grow in the absence of the gene. Therefore, by deleting an arbitrary gene in a viral genome and detecting the growth of the virus, it is possible to determine whether the gene is an essential gene or a non-essential gene.

As used herein, the term "wild strain" in relation to varicella-zoster virus refers to a varicella-zoster virus strain which is not artificially modified and is isolated from nature. An example of a wild strain includes, but is not limited to, Dumas strain identified by Davison, A.J. and Scott, J. E. (J. Gen. Virol. 67(Pt 9), 1759-1816 (1986) . The nucleic acid sequence of Dumas strain is set forth in SEQ ID NO.: 5. The number of each ORF and the site thereof in the Dumas strain are described below.

| ORF Name | Reading frame direction | Site on genome | Number of amino acid residues |
|---|---|---|---|
| ORF1 | 3'→5' direction | 589 to 915 | amino acid 1-108 |
| ORF2 | 5'→3' direction | 1134 to 1850 | amino acid 1-238 |
| ORF3 | 3'→5' direction | 1908 to 2447 | amino acid 1-179 |
| ORF4 | 3'→5' direction | 2783 to 4141 | amino acid 1-452 |
| ORF5 | 3'→5' direction | 4252 to 5274 | amino acid 1-340 |
| ORF6 | 3'→5' direction | 5326 to 8577 | amino acid 1-1083 |
| ORF7 | 5'→3' direction | 8607 to 9386 | amino acid 1-259 |
| ORF8 | 3'→5' direction | 9477 to 10667 | amino acid 1-396 |
| ORF9 | 5'→3' direction | 11009 to 11917 | amino acid 1-302 |
| ORF9A | 5'→3' direction | 10642 to 10902 | amino acid 1-87 |
| ORF10 | 5'→3' direction | 12160 to 13392 | amino acid 1-410 |
| ORF11 | 5'→3' direction | 13590 to 16049 | amino acid 1-819 |
| ORF12 | 5'→3' direction | 16214 to 18199 | amino acid 1-661 |
| ORF13 | 5'→3' direction | 18441 to 19346 | amino acid 1-301 |
| ORF14 | 3'→5' direction | 19431 to 21113 | amino acid 1-560 |
| ORF15 | 3'→5' direction | 21258 to 22478 | amino acid 1-406 |
| ORF16 | 3'→5' direction | 22568 to 23794 | amino acid 1-408 |
| ORF17 | 5'→3' direction | 24149 to 25516 | amino acid 1-455 |
| ORF18 | 3'→5' direction | 25573 to 26493 | amino acid 1-306 |
| ORF19 | 3'→5' direction | 26518 to 28845 | amino acid 1-775 |
| ORF20 | 3'→5' direction | 29024 to 30475 | amino acid 1-483 |
| ORF21 | 5'→3' direction | 30759 to 33875 | amino acid 1 -1038 |
| ORF22 | 5'→3' direction | 34083 to 42374 | amino acid 1-2763 |
| ORF23 | 3'→5' direction | 42431 to 43138 | amino acid 1-235 |
| ORF24 | 3'→5' direction | 43212 to 44021 | amino acid 1-269 |
| ORF25 | 3'→5' direction | 44148 to 44618 | amino acid 1-156 |
| ORF26 | 5'→3' direction | 44506 to 46263 | amino acid 1-585 |
| ORF27 | 5'→3' direction | 46127 to 47128 | amino acid 1-333 |
| ORF28 | 3'→5' direction | 47052 to 50636 | amino acid 1-1194 |
| ORF29 | 5'→3' direction | 50857 to 54471 | amino acid 1-1204 |
| ORF30 | 5'→3' direction | 54651 to 56963 | amino acid 1-770 |
| ORF31 | 5'→3' direction | 57008 to 59614 | amino acid 1-868 |
| ORF32 | 5'→3' direction | 59766 to 60197 | amino acid 1-143 |
| ORF33 | 3'→5' direction | 60321 to 62138 | amino acid 1-605 |
| ORF33.5 | 3'→5' direction | 60321 to 61229 | amino acid 1-301 |
| ORF34 | 3'→5' direction | 62171 to 63910 | amino acid 1-579 |
| ORF35 | 3'→5' direction | 63977 to 64753 | amino acid 1-258 |
| ORF36 | 5'→3' direction | 64807 to 65832 | amino acid 1-341 |
| ORF37 | 5'→3' direction | 66074 to 68599 | amino acid 1-841 |
| ORF38 | 3'→5' direction | 68668 to 70293 | amino acid 1-541 |
| ORF39 | 5'→3' direction | 70633 to 71355 | amino acid 1-240 |
| ORF40 | 5'→3' direction | 71540 to 75730 | amino acid 1-1396 |
| ORF41 | 5'→3' direction | 75847 to 76797 | amino acid 1-316 |
| ORF42+45 | 3'→5' direction | 76851 to 78038 and 81538 to 82593 | amino acid 1-747 |
| ORF43 | 5'→3' direction | 78170 to 80200 | amino acid 1-676 |
| ORF44 | 5'→3' direction | 80360 to 81451 | amino acid 1-363 |
| ORF46 | 5'→3' direction | 82719 to 83318 | amino acid 1-199 |
| ORF47 | 5'→3' direction | 83168 to 84700 | amino acid 1-510 |
| ORF48 | 5'→3' direction | 84667 to 86322 | amino acid 1-551 |
| ORF49 | 5'→3' direction | 86226 to 86471 | amino acid 1-81 |
| ORF50 | 3'→5' direction | 86575 to 87882 | amino acid 1-435 |
| ORF51 | 5'→3' direction | 87881 to 90388 | amino acid 1-835 |
| ORF52 | 5'→3' direction | 90493 to 92808 | amino acid 1-771 |
| ORF53 | 3'→5' direction | 92855 to 93850 | amino acid 1-331 |
| ORF54 | 3'→5' direction | 93675 to 95984 | amino acid 1-769 |
| ORF55 | 5'→3' direction | 95996 to 98641 | amino acid 1-881 |
| ORF56 | 5'→3' direction | 98568 to 99302 | amino acid 1-244 |
| ORF57 | 3'→5' direction | 99411 to 99626 | amino acid 1-71 |
| ORF58 | 3'→5' direction | 99607 to 100272 | amino acid 1-221 |
| ORF59 1-305 | 3'→5' direction | 100302 to 101219 | amino acid |
| ORF60 1-159 | 3'→5' direction | 101170 to 101649 | amino acid |
| ORF61 1-467 | 3'→5' direction | 103082 to 104485 | amino acid |
| ORF62 1-1310 | 3'→5' direction | 105201 to 109133 | amino acid |
| ORF63 1-278 | 5'→3' direction | 110581 to 111417 | amino acid |
| ORF64 1-180 | 5'→3' direction | 111565 to 112107 | amino acid |
| ORF65 1-102 | 3'→5' direction | 112332 to 112640 | amino acid |
| ORF66 1-393 | 5'→3' direction | 113037 to 114218 | amino acid |
| ORF67 1-354 | 5'→3' direction | 114496 to 115560 | amino acid |
| ORF68 1-623 | 5'→3' direction | 115808 to 117679 | amino acid |
| ORF69 1-180 | 3'→5' direction | 117790 to 118332 | amino acid |
| ORF70 1-278 | 3'→5' direction | 118480 to 119316 | amino acid |
| ORF71 1-1310 | 5→3' direction | 120764 to 124696 | amino acid |

In the above-described table, "5'→3' direction" indicates that the ORF has the same direction as that of the nucleic acid sequence of SEQ ID NO.: 5. "3'→5' direction" indicates that the ORF has a reverse direction with respect to that of the nucleic acid sequence of SEQ ID NO.: 5. By identifying a sequence homologous to the nucleic acid sequence and/or the amino acid sequence of the ORF, those skilled in the art can easily identify the ORF in the genome of a strain other than Dumas strain.

As used herein, the term "mutant strain" refers to a varicella-zoster virus strain which has a mutation due to mutagenesis, multiple subculturings or the like. Mutagenesis of a varicella-zoster virus strain may be either random mutagenesis or site-specific mutagenesis.

The terms "attenuated virus" as used herein is a type of a virus mutant strain and refer to the one that has lower virulence than wild strain. Two methods for deciding whether the virulence of a virus mutant strain is lower than that of wild strain or not (that is, the method for examining the pathogenicity of varicella-zoster virus) have been established.

As a method using an animal model, the method for evaluating the pathogenicity by producing a severe combined immunodeficient (SCID) mouse to which human skin is transplanted, and then, to infect the mouse with varicella-zoster virus is well-known (J. Viroll. 1998 Feb; 72(2): 965-74,).

On the other hand, as a method for evaluating the pathogenicity *in vitro,* the method for observing CPE (cytopathic effect) of melanoma after culturing for 7-8 days where a monolayer culture human melanoma is inserted to the lower layer and a cord-blood mononuclear cell (CBMC) which is infected with varicella-zoster cell virus is inserted to the upper layer of a two-layer well separated by a trans-well of pore size 3 µm, is also well-known (J. Virol. 2000 Feb; 74(4) : 1864-70).

Although it is not the method for the pathogenicity directly from the study of the present inventors (J Virol. 2002 Nov; 76 (22) : 11447-59), which is understood to indicate that there are close relationships between the pathogenicity and the proliferation of a virus, it is also possible to evaluate the pathogenicity indirectly by examining the proliferation of cell-to-cell employing the infectious center assay.

The method for attenuating a virus artificially is well known. For example, a varicella-zoster virus comprises at least the base substitutions of the following (a)-(d) in the gene 62 in SEQ ID NO.5:
(a) base substitution at position 2110 for G;
(b) base substitution at position 3100 for G;
(c) base substitution at position 3818 for C; and
(d) base substitution at position 4006 for G,
   and comprises at least a base substitution at position 5745 for G, in the gene 6 in SEQ ID NO. 8 is available as an attenuated virus.

In addition to the base substitutions of (a)-(d), instead of employing the above-mentioned varicella-zoster virus, an attenuated varicella virus which comprises at least one or more base substitutions of following (e)-(g):
(e) base substitution at position 1251 for G;
(f) base substitution at position 2226 for G; and
(g) base substitution at position 3657 for G,
   is available.

In addition to one or more base substitutions of (a)-(g), instead of employing the above-mentioned varicella-zoster virus, an attenuated varicella virus which comprises at least one or more base substitutions of the following (h)-(o):
(h) base substitution at position 162 for C;
(i) base substitution at position 225 for C;
(j) base substitution at position 523 for C;
(k) base substitution at position 1565 for C;
(1) base substitution at position 1763 for C;
(m) base substitution at position 2652 for C;
(n) base substitution at position 4052 for C; and
(o) base substitution at position 4193 for C,
   is available.

Alternatively, as an "attenuated virus", a virus which comprises at least one or more base substitutions selected from the following group in the gene 62:
(a) base substitution at position 2110 for G;
(b) base substitution at position 3100 for G;
(c) base substitution at position 3818 for C;
(d) base substitution at position 4006 for G;
(e) base substitution at position 1251 for G;
(f) base substitution at position 2226 for G;
(g) base substitution at position 3657 for G;
(h) base substitution at position 162 for C;
(i) base substitution at position 225 for C;
(j) base substitution at position 523 for C;
(k) base substitution at position 1565 for C;
(1) base substitution at position 1763 for C;
(m) base substitution at position 2652 for C;
(n) base substitution at position 4052 for C; and
(o) base substitution at position 4193 for C,
   can be employed.

The terms "protein", "polypeptide", "oligopeptide" and "peptide" as used herein have the same meaning and refer to an amino acid polymer having any length.

The terms "polynucleotide", "oligonucleotide", and "nucleic acid" as used herein have the same meaning and refer to a nucleotide polymer having any length. Unless otherwise indicated, a particular nucleic acid sequence also implicitly encompasses conservatively-modified variants thereof (e.g. degenerate codon substitutions) and complementary sequences as well as the sequence explicitly indicated. Specifically, degenerate codon substitutions may be produced by generating sequences in which the third position of one or more selected (or all) codons is substituted with mixed-base and/or deoxyinosine residues (Batzer et al., Nucleic Acid Res. 19:5081(1991); Ohtsuka et al., J. Biol. Chem. 260:2605-2608 (1985); Rossolini et al., Mol. Cell. Probes 8:91-98(1994)).

As used herein, the term "gene" refers to an element defining a genetic trait. A gene is typically arranged in a given sequence on a chromosome. A gene which defines the primary structure of a protein is called a structural gene. A gene which regulates the expression of a structural gene is called a regulatory gene. As used herein, "gene" may refer to "polynucleotide", "oligonucleotide", "nucleic acid", and "nucleic acid molecule" and/or "protein", "polypeptide", "oligopeptide" and "peptide". As used herein, the term "open reading frame" or "ORF" in relation to a gene, refers to a reading frame which is one of three frames obtained by sectioning the base sequence of a gene at intervals of three bases, and has a start codon and a certain length without a stop codon appearing partway, and has the possibility of actually coding a protein. The entire base sequence of the genome of varicella-zoster virus has been determined, identifying at least 71 genes. Each of the genes is known to have an open reading frame (ORF).

As used herein, the term "region within an ORF" in relation to a gene in a varicella-zoster virus genome, refers to a region in which there are bases constituting the ORF in the gene within the varicella-zoster virus genome.

As used herein, the term "region flanking an ORF" in relation to a gene in a varicella-zoster virus genome, refers to a region in which there are bases existing in the vicinity of the ORF in the gene within the varicella-zoster virus genome, and which does not correspond to a region within the ORF of the gene or other genes.

As used herein, the term "homology" of a gene refers to the proportion of identity between two or more gene sequences. Therefore, the greater the homology between two given genes, the greater the identity or similarity between their sequences. Whether or not two genes have homology is determined by comparing their sequences directly or by a hybridization method under stringent conditions. When two gene sequences are directly compared with each other, these genes have homology if the DNA sequences of the genes have representatively at least 50% identity, preferably at least 70% identity, more preferably at least 80%, 90%, 95%, 96%, 97%, 98%, or 99% identity with each other.

Similarity comparison and homology calculation of base sequences are herein performed using BLAST (sequence analyzing tool) with the default parameters.

As used herein, the term "expression" of a gene, a polynucleotide, a polypeptide, or the like, indicates that the gene or the like is affected by a predetermined action *in vivo* to be changed into another form. Preferably, the term "expression" indicates that genes, polynucleotides, or the like are transcribed and translated into polypeptides. In one embodiment of the present invention, genes may be transcribed into mRNA. More preferably, these polypeptides may have post-translational processing modifications.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

As used herein, the term "fragment" refers to a polypeptide or polynucleotide having a sequence length ranging from 1 to n-1 with respect to the full length of the reference polypeptide or polynucleotide (of length n). The length of the fragment can be appropriately changed depending on the purpose. For example, in the case of polypeptides, the lower limit of the length of the fragment includes 3, 4, 5, 6, 7, 8, 9, 10, 15, 20, 25, 30, 40, 50 or more nucleotides. Lengths represented by integers which are not herein specified (e.g., 11 and the like) may be appropriate as a lower limit. For example, in the case of polynucleotides, the lower limit of the length of the fragment includes 5, 6, 7, 8, 9, 10, 15, 20, 25, 30, 40, 50, 75, 100, 200, 300, 400, 500, 600, 600, 700, 800, 900, 1000 or more nucleotides. Lengths represented by integers which are not herein specified (e.g., 11 and the like) may be appropriate as a lower limit.

A polypeptide encoded by a gene in a BAC vector may have at least one (e.g., one or several) amino acid substitution, addition, and/or deletion or at least one sugar chain substitution, addition, and/or deletion as long as they have substantially the same function as that of a corresponding naturally-occurring polypeptide.

As used herein, the term "sugar chain" refers to a compound which is made up of a series of at least one sugar unit (a monosaccharide and/or its derivative). When two or more sugars unit is linked, the sugars unit are joined by dehydrocondensation due to glycosidic bonds. Examples of such a sugar chain include, but are not limited to, polysaccharides contained in organisms (glucose, galactose, mannose, fucose, xylose, N-acetylglucosamine, N-acetylgalactosamine, sialic acid, and complexes and derivates thereof), and degraded polysaccharides, sugar chains degraded or induced from complex biological molecules (e.g., glycoproteins, proteoglycan, glycosaminoglycan, glycolipids, etc.), and the like. Therefore, theterm"sugar chain" may be herein used interchangeably with "polysaccharide", "carbohydrate", and "hydrocarbon". Unless otherwise specified, the term "sugar chain" as used herein includes both a sugar chain and a sugar chain-containing substance.

It is well known that if a given amino acid is substituted with another amino acid having a similar hydrophobicity index, the resultant protein may still have a biological function similar to that of the original protein (e.g., a protein having an equivalent enzymatic activity). For such an amino acid substitution, the hydrophobicity index is preferably within ±2, more preferably within ±1, and even more preferably within ±0.5. It is understood in the art that such an amino acid substitution based on hydrophobicity is efficient. A hydrophilicity index is also useful for modification of an amino acid sequence of the present invention. As described in US Patent No. 4, 554, 101, amino acid residues are given the following hydrophilicity indices: arginine (+3.0); lysine (+3.0); aspartic acid (+3.0±1); glutamic acid (+3.0±1); serine (+0.3); asparagine (+0.2); glutamine (+0.2); glycine (0); threonine (-0.4); proline (-0.5±1); alanine (-0.5);histidine (-0.5); cysteine (-1.0); methionine (-1.3);valine (-1.5);leucine (-1.8); isoleucine (-1.8); tyrosine (-2.3); phenylalanine (-2.5); and tryptophan (-3.4). It is understood that an amino acid may be substituted with another amino acid which has a similar hydrophilicity index and can still provide a biological equivalent. For such an amino acid substitution, the hydrophilicity index is preferably within ±2,more preferably ±1, and even more preferably ±0.5.

The term "conservative substitution" as used herein refers to amino acid substitution in which a substituted amino acid and a substituting amino acid have similar hydrophilicity indices or/and hydrophobicity indices. For example, conservative substitution is carried out between amino acids having a hydrophilicity or hydrophobicity index of within ±2, preferably within ±1, and more preferably within ±0.5. Examples of conservative substitution include, but are not limited to, substitutions within each of the following residue pairs: arginine and lysine; glutamic acid and aspartic acid; serine and threonine; glutamine and asparagine; and valine, leucine, and isoleucine, which are well known to those skilled in the art.

As used herein, the term "variant" refers to a substance, such as a polypeptide, polynucleotide, or the like, which differs partially from the original substance. Examples of such a variant include a substitution variant, an addition variant, a deletion variant, a truncated variant, an allelic variant, and the like. Examples of such a variant include, but are not limited to, a nucleotide or polypeptide having one or several substitutions, additions and/or deletions or a nucleotide or polypeptide having at least one substitution, addition and/or deletion. The term "allele" as used herein refers to a genetic variant located at a locus identical to a corresponding gene, where the two genes aredistinguishedfromeachother. Therefore, the term "allelic variant" as used herein refers to a variant which has an allelic relationship with a given gene. Such an allelic variant ordinarily has a sequence the same as or highly similar to that of the corresponding allele, and ordinarily has almost the same biological activity, though it rarely has different biological activity. The term "species homolog" or "homolog" as used herein refers to one that has an amino acid or nucleotide homology with a given gene in a given species (preferably at least 60% homology, more preferably at least 80%, at least 85%, at least 90%, and at least 95% homology). A method for obtaining such a species homolog is clearly understood from the description of the present specification. The term "ortholog" (also called orthologous genes) refers to genes in different species derived from a common ancestry (due to speciation). For example, in the case of the hemoglobin gene family having multigene structure, human and mouse α-hemoglobin genes are orthologs, while the human α-hemoglobin gene and the human β-hemoglobin gene are paralogs (genes arising from gene duplication). Orthologs are useful for estimation of molecular phylogenetic trees. Usually, orthologs in different species may have a function similar to that of the original species. Therefore, orthologs of the present invention may be useful in the present invention.

As used herein, the term "conservative (or conservatively modified) variant" applies to both amino acid and nucleic acid sequences. With respect to particular nucleic acid sequences, conservatively modified variants refer to those nucleic acids which encode identical or essentially identical amino acid sequences. Because of the degeneracy of the genetic code, a large number of functionally identical nucleic acids encode any given protein. For example, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations" which represent one species of conservatively modified variation. Every nucleic acid sequence herein which encodes a polypeptide also describes every possible silent variation of the nucleic acid. Those skilled in the art will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid which encodes a polypeptide is implicit in each described sequence. Preferably, such modification may be performed while avoiding substitution of cysteine which is an amino acid capable of largely affecting the higher-order structure of a polypeptide.
In order to prepare a BAC vector containing a gene encoding a functionally equivalent polypeptide, amino acid additions, deletions, or modifications can be performed in addition to amino acid substitutions. Amino acid substitution(s) refers to the replacement of at least one amino acid of an original peptide chain with different amino acids, such as the replacement of 1 to 10 amino acids, preferably 1 to 5 amino acids, and more preferably 1 to 3 amino acids with different amino acids. Amino acid addition(s) refers to the addition of at least one amino acid to an original peptide chain, such as the addition of 1 to 10 amino acids, preferably 1 to 5 amino acids, and more preferably 1 to 3 amino acids to an original peptide chain. Amino acid deletion(s) refers to the deletion of at least one amino acid, such as the deletion of 1 to 10 amino acids, preferably 1 to 5 amino acids, and more preferably 1 to 3 amino acids. Amino acid modification includes, but are not limited to, amidation, carboxylation, sulfation, halogenation, truncation, lipidation, alkylation, glycosylation, phosphorylation, hydroxylation, acylation (e.g., acetylation), and the like. Amino acids to be substituted or added may be naturally-occurring or nonnaturally-occurring amino acids, or amino acid analogs. Naturally-occurring amino acids are preferable.

As used herein, a nucleic acid form of a polypeptide refers to a nucleic acid molecule capable of expressing a protein form of the polypeptide. This nucleic acid molecule may have a nucleic acid sequence, a part of which is deleted or substituted with another base, or alternatively, into which another nucleic acid sequence is inserted, as long as an expressed polypeptide has substantially the same activity as that of a naturally occurring polypeptide. Alternatively, another nucleic acid may be linked to the 5' end and/or the 3' end of the nucleic acid molecule. The nucleic acid molecule may be a nucleic acid molecule which is hybridizable to a gene encoding a polypeptide under stringent conditions and encodes a polypeptide having substantially the same function as that polypeptide. Such a gene is known in the art and is available in the present invention.

Such a nucleic acid can be obtained by a well known PCR technique, or alternatively, can be chemically synthesized. These methods may be combined with, for example, site-specific mutagenesis, hybridization, or the like.

As used herein, the term "substitution, addition or deletion" for a polypeptide or a polynucleotide refers to the substitution, addition or deletion of an amino acid or its substitute, or a nucleotide or its substitute, with respect to the original polypeptide or polynucleotide, respectively. This is achieved by techniques well-known in the art, including a site-specific mutagenesis technique, and the like. A polypeptide or a polynucleotide may have any number (>0) of substitutions, additions, or deletions. The number can be as large as a variant having such a number of substitutions, additions or deletions which maintains an intended function. For example, such a number may be one or several, and preferably within 20% or 10% of the full length, or no more than 100, no more than 50, no more than 25, or the like.

The structure of polymers (e.g., polypeptide structure) may be described at various levels. This structure is generally described in, for example, Alberts et al., Molecular Biology of the Cell (3rd Ed., 1994), and Cantor and Schimmel, Biophysical Chemistry Part I: The Conformation of Biological Macromolecules (1980). General molecular biological techniques available in the present invention can be easily carried out by the those skilled in the art by referencing Ausubel F. A. et al. eds. (1988), Current Protocols in Molecular Biology, Wiley, New York, NY; SambrookJ. et al., (1987) Molecular Cloning: ALaboratory Manual, 2nd Ed., Cold Spring Harbor Laboratory Press, Cold Spring Harbor, NY, or the like.

When mentioning genes in the present specification, "vector" refers to an agent which can transfer a polynucleotide sequence of interest to a target cell. Examples of such a vector include vectors which are capable of self replication or capable of being incorporated into a chromosome within host cells (e.g., prokaryotic cells, yeast, animal cells, plant cells, insect cells, whole animals, and whole plants), and contain a promoter at a site suitable for transcription of a polynucleotide of the present invention.

The term "BAC vector" refers to a plasmid which is produced using F plasmid of E. *coli* and a vector which can stably maintain and grow a large size DNA fragment of about 300 kb or more in bacteria, such as E. *coli* and the like. The BAC vector contains at least a region essential for the replication of the BAC vector. Examples of such a region essential for replication include, but are not limited to, the replication origin of F plasmid (oriS) and variants thereof.

As used herein, the term "BAC vector sequence" refers to a sequence comprising a sequence essential for the function of a BAC vector. Optionally, the BAC vector sequence may further comprise a "recombinant protein-dependent recombinant sequence" and/or a "selectable marker".

As used herein, the term "recombinant" in relation to nucleic acid is used interchangeably with the term "homologous recombination", and indicates that two different homologous nucleic acid molecules encounter each other, crossover occurs, and a new combination of nucleic acid is generated. As used herein, homologous recombination includes both "recombinant protein-dependent recombination" and "recombinant protein-independent recombination". The term "recombinant protein-dependent recombination" refers to homologous recombination which occurs in the presence of a recombinant protein, but not in the absence of a recombinant protein. The term "recombinant protein-independent recombination" refers to homologous recombination which occurs irrespective of the presence or absence of a recombinant protein. As used herein, the term "recombinant protein-dependent recombinant sequence" refers to a sequence which causes recombinant protein-dependent recombination. The term "recombinant protein-independent recombinant sequence" refers to a sequence which causes recombinant protein-independent recombination. The recombinant protein-dependent recombinant sequence causes recombination in the presence of a recombinant protein, but not in the absence of a recombinant protein. A recombinant protein preferably acts specifically on a recombinant protein-dependent recombinant sequence, and does not act on sequences other than the recombinant protein-dependent recombinant sequence.

Examples of representative pairs of a recombinant protein-dependent recombinant sequence and a recombinant protein include, but are not limited to: a combination of a bacteriophage P1-derived loxP (locus of crossover of P1) sequence and a Cre (cyclization recombination) protein, a combination of Flp protein and FRT site, a combination of ϕC31 and attB or attP (Thorpe, Helena M.; Wilson, Stuart E.; Smith, Margaret C.M., Control of directionality in the site-specific recombination system of the Streptomyces phage ϕC31., Molecular Microbiology (2000), 38(2), 232-241.), a combination of resolvase and res site(Sadowski P., Site-specific recombinases: changing partners and doing the twist, J. Bacteriol., February 1986; 165(2) 341-7) (generally, Sauer B., Site-specific recombination: developments and applications., Curr. Opin. Biotechnol., 1994 Oct; 5(5): 521-7).

As used herein, the term "selectable marker" refers to a gene which functions as an index for selection of a host cell containing a BAC vector. Examples of a selectable marker include, but are not limited to, fluorescent markers, luminiscent markers, and drug selectable markers. An example of a "fluorescent marker" is, but is not limited to, a gene encoding a fluorescent protein, such as a green fluorescent protein (GFP). An example of a "luminiscent marker" is, but is not limited to, a gene encoding a luminescent protein, such as luciferase. An example of a "drug selectable marker" is, but is not limited to, a gene encoding a protein selected from the group consisting of: dihydrofolate reductase gene, glutamine synthase gene, aspartic acid transaminase, metallothionein (MT), adenosine deaminase (ADA), adenosine deaminase (AMPD1, 2), xanthine-guanine-phosphoribosyl transf erase, UMP synthase, P-glycoprotein, asparagine synthase, and ornithine decarboxylase. Examples of a combination of a drug selectable marker and a drug include: a combination of dihydrofolate reductase gene (DHFR) and methotrexate (MTX), a combination of glutamine synthase (GS) gene and methionine sulfoximine (Msx), a combination of aspartic acid transaminase (CAD) gene and N-phosphonacetyl-L-aspartate) (PALA), a combination of MT gene and cadmium (Cd2⁺), a combination of adenosine deaminase (ADA) gene and adenosine, alanosine, or 2'-deoxycoformycin, a combination of adenosine deaminase (AMPD1, 2) gene and adenine, azaserine, or coformycin, a combination of xanthine-guanine-phosphoribosyltransferase gene and mycophenolic acid, a combination of UMP synthase gene and 6-azaulysine or pyrazofuran, a combination of P-glycoprotein (P-gp, MDR) gene and multiple drugs, a combination of asparagine synthase (AS) gene and β-aspartyl hydroxamic acid or albizziinn, and a combination of ornithine decarboxylase (ODC) gene and α-difluoromethyl-ornithine (DFMO).

As used herein, the term "expression vector" refers to a nucleic acid sequence comprising a structural gene and a promoter for regulating expression thereof, and in addition, various regulatory elements in a state that allows them to operate within host cells. The regulatory element may include, preferably, terminators, selectable markers such as drug-resistance genes (e.g., a kanamycin resistance gene, a hygromycin resistance gene, etc.), and enhancers. It is well known to those skilled in the art that the type of an organism (e.g., a plant) expression vector and the type of a regulatory element may vary depending on the host cell. In the case of plants, a plant expression vector for use in the present invention may further have a T-DNA region. A T-DNA region enhances the efficiency of gene transfer, especially when a plant is transformed using Agrobacterium.

As used herein, the term "recombinant vector" refers to a vector which can transfer a polynucleotide sequence of interest to a target cell. Examples of such a vector include vectors which are capable of self replication or capable of being incorporated into a chromosome within host cells (e.g., prokaryotic cells, yeast, animal cells, plant cells, insect cells, whole animals, and whole plants), and contain a promoter at a site suitable for transcription of a polynucleotide of the present invention.

As used herein, the term "terminator" refers to a sequence which is located downstream of a protein-encoding region of a gene and which is involved in the termination of transcription when DNA is transcribed into mRNA, and the addition of a poly A sequence. It is known that a terminator contributes to the stability of mRNA, and has an influence on the amount of gene expression. Examples of a terminator include, but are not limited to, terminators derived from mammals, the CaMV35S terminator, the terminator of the nopaline synthase gene (Tnos), the terminator of the tobacco PR1a gene, and the like.

As used herein, the term "promoter" refers to a base sequence which determines the initiation site of transcription of a gene and is the region in the ORF of DNA which directly regulates the frequency of transcription. Transcription is started by RNA polymerase binding to a promoter. A promoter region is usually located within about 2 kbp upstream of the first exon of a putative protein coding region. Therefore, it is possible to estimate a promoter region by predicting a protein coding region in a genomic base sequence using DNA analysis software. A putative promoter region is usually located upstream of a structural gene, but depending on the structural gene, i.e., a putative promoter region may be located downstream of a structural gene. Preferably, a putative promoter region is located within about 2 kbp upstream of the translation initiation site of the first exon.

As used herein, the term "constitutive" for expression of a promoter of the present invention refers to a character of the promoter that the promoter is expressed in a substantially constant amount in all tissues of an organism no matter whether the growth stage of the organism is a juvenile phase or a mature phase. Specifically, when Northern blotting analysis is performed under the same conditions as those described in examples of the present specification, expression is considered to be constitutive according to the definition of the present invention if substantially the same amount of expression is observed at the same or corresponding site at any time (e.g., two or more time points (e.g., day 5 and day 15)), for example. Constitutive promoters are considered to play a role in maintaining the homeostasis of organisms in a normal growth environment. These characters can be determined by extracting RNA from any portion of an organism and analyzing the expression amount of the RNA by Northern blotting or quantitating expressed proteins by Western blotting.

An "enhancer" may be used so as to enhance the expression efficiency of a gene of interest. When used in animals, an enhancer region containing an upstream sequence within the SV40 promoter is preferable. One or more enhancers may be used, or no enhancer may be used.

As used herein, the term "operatively linked" indicates that a desired sequence is located such that expression (operation) thereof is under control of a transcription and translation regulatory sequence (e.g., a promoter, an enhancer, and the like) or a translation regulatory sequence. In order for a promoter to be operatively linked to a gene, typically, the promoter is located immediately upstream of the gene. A promoter is not necessarily adjacent to a structural gene.

As used herein, the terms "transformation", "transduction" and "transfection" are used interchangeably unless otherwise mentioned, and refers to introduction of a nucleic acid into host cells. As a transformation method, any technique for introducing DNA into host cells can be used, including various well-known techniques, such as, for example, the electroporation method, the particle gun method (gene gun), the calcium phosphate method, and the like.

As used herein, the term "transformant" refers to the whole or a part of an organism, such as a cell, which is produced by transformation. Examples of a transformant include prokaryotic cells, yeast, animal cells, plant cells, insect cells and the like. Transformants may be referred to as transformed cells, transformed tissue, transformed hosts, or the like, depending on the subject. As used herein, all of the forms are encompassed, however, a particular form may be specified in a particular context.

Examples of prokaryotic cells include prokaryotic cells of the genera *Escherichia, Serratia, Bacillus, Brevibacterium, Corynebacterium, Microbacterium, Pseudomonas,* and the like, e.g., *Escherichia coli* XL1-Blue, *Escherichia coli*XL2-Blue, *Escherichia coli* DH1, *Escherichia coli* MC1000, *Escherichia coli* KY3276, *Escherichia coli* W1485, *Escherichia coli* JM109, *Escherichia coli* HB101, *Escherichia coli* No.49, *Escherichia coli* W3110, *Escherichia coli* NY49, *Escherichia coli* BL21(DE3), *Escherichia coli* BL21(DE3)pLysS, *Escherichia coli* HMS174(DE3), *Escherichia coli* HMS174(DE3)pLysS, *Serratia ficaria, Serratia fonticola, Serratia liquefaciens, Serratia* marcescens, *Bacillus subtilis, Bacillus amyloliquefaciens,* Brevibacteriumammmoniagenes, Brevibacteriumimmariophilum ATCC14068, *Brevibacterium saccharolyticum* ATCC14066, *Corynebacterium glutamicum* ATCC13032, *Corynebacterium glutamicum* ATCC14067, *Corynebacterium glutamicum* ATCC13869, *Corynebacterium* acetoacidophilum ATCC13870, *Microbacterium ammoniaphilum* ATCC15354, *Pseudomonas* sp.D-0110, and the like.

Examples of animal cells include human MRC-5 cells, human HEL cells, human WI-38 cells, mouse myeloma cells, rat myeloma cells, human myeloma cells, mouse hybridoma cells, CHO cells derived from chinese hamster, BHK cells, African green monkey kidney cells, human leukemia cells, HBT5637 (Japanese Laid-Open Publication No. 63-299), human colon cancer cell strains. Mouse myeloma cells include ps20, NSO, and the like. Rat myeloma cells include YB2/0, and the like. Human fetus kidney cells includes HEK293 (ATCC: CRL-1573), and the like. Human leukemia cells include BALL-1, and the like. African green monkey kidney cells include COS-1, COS-7, vero cell and the like. Human colon cancer cell strains include HCT-15, and the like.

The term "animal" is used herein in its broadest sense and refers to vertebrates and invertebrates (e.g., arthropods). Examples of animals include, but are not limited to, any of the class *Mammalia,* the class *Aves,* the class *Reptilia,* the class *Amphibia,* the class *Pisces,* the class *Insecta,* the class *Vermes,* and the like.

As used herein, the term "tissue" in relation to organisms refers to an aggregate of cells having substantially the same function. Therefore, a tissue may be a part of an organ. Organs usually have cells having the same function, and may have coexisting cells having slightly different functions. Therefore, as used herein, tissues may have various kinds of cells as long as a certain property is shared by the cells.

As used herein, the term "organ" refers to a structure which has a single independent form and in which one or more tissues are associated together to perform a specific function. In plants, examples of organs include, but are not limited to, callus, root, stem, trunk, leaf, flower, seed, embryo bud, embryo, fruit, and the like. In animals, examples of organs include, but are not limited to, stomach, liver, intestine, pancreas, lung, airway, nose, heart, artery, vein, lymph node (lymphatic system), thymus, ovary, eye, ear, tongue, skin, and the like.

As used herein, the term "transgenic" refers to incorporation of a specific gene into an organism (e.g., plants or animals (mice, etc.)) or such an organism having an incorporated gene.

When organisms of the present invention are animals, the transgenic organisms can be produced by a microinjection method (a trace amount injection method), a viral vector method, an embryonic stem (ES) cell method, a sperm vector method, a chromosome fragment introducing method (transsomic method), an episome method, or the like. These transgenic animal producing techniques are well known in the art.

As used herein, the term "screening" refers to selection of a substance, a host cell, a virus, or the like having a given specific property of interest from a number of candidates using a specific operation/evaluation method. It will be understood that the present invention encompasses viruses having desired activity obtained by screening.

As used herein, the terms "chip" or "microchip" are used interchangeably to refer to a micro integrated circuit which has versatile functions and constitutes a portion of a system. Examples of a chip include, but are not limited to, DNA chips, protein chips, and the like.

As used herein, the term "array" refers to a substrate (e.g., a chip, etc.) which has a pattern of a composition containing at least one (e.g., 1000 or more, etc.) target substances (e.g., DNA, proteins, cells, etc.), which are arrayed. Among arrays, patterned substrates having a small size (e.g., 10x10 mm, etc.) are particularly referred to as microarrays. The terms "microarray" and "array" are used interchangeably. Therefore, a patterned substrate having a larger size than that which is described above may be referred to as a microarray. For example, an array comprises a set of desired transfection mixtures fixed to a solid phase surface or a film thereof. An array preferably comprises at least 10² antibodies of the same or different types, more preferably at least 10³, even more preferably at least 10⁴, and still even more preferably at least 10⁵. These antibodies are placed on a surface of up to 125x80 mm, more preferably 10×10 mm. An array includes, but is not limited to, a 96-well microtiter plate, a 384-well microtiter plate, a microtiter plate the size of a glass slide, and the like. A composition to be fixed may contain one or a plurality of types of target substances. Such a number of target substance types may be in the range of from one to the number of spots, including, without limitation, about 10, about 100, about 500, and about 1,000.

As described above, any number of target substances (e.g., biomolecular, such as cells) may be provided on a solid phase surface or film, typically including no more than 10⁸ biological molecules per substrate, in another embodiment no more than 10⁷ biological molecules, no more than 10⁶ biological molecules, no more than 10⁵ biological molecules, no more than 10⁴ biological molecules, no more than 10³ biological molecules, or no more than 10² biological molecules. A composition containing more than 10⁸ biological molecule target substances may be provided on a substrate. In these cases, the size of a substrate is preferably small. Particularly, the size of a spot of a composition containing target substances (e.g., such as cells) may be as small as the size of a single biological molecule (e.g., 1 to 2 nm order). In some cases, the minimum area of a substrate may be determined based on the number of biological molecules on a substrate.

"Spots" of biological molecules may be provided on an array. As used herein, the term "spot" refers to a certain set of compositions containing target substances. As used herein, the term "spotting" refers to an act of preparing a spot of a composition containing a certain target substance on a substrate or plate. Spotting may be performed by any method, for example, pipetting or the like, or alternatively, using an automatic device. These methods are well known in the art.

As used herein, the term "address" refers to a unique position on a substrate, which may be distinguished from other unique positions. Addresses are appropriately associated with spots. Addresses can have any distinguishable shape such that substances at each address may be distinguished from substances at other addresses (e. g. , optically). A shape defining an address may be, for example, without limitation, a circle, an ellipse, a square, a rectangle, or an irregular shape. Therefore, the term "address" is used to indicate an abstract concept, while the term "spot" is used to indicate a specific concept. Unless it is necessary to distinguish them from each other, the terms "address" and "spot" may be herein used interchangeably.

The size of each address particularly depends on the size of the substrate, the number of addresses on the substrate, the amount of a composition containing target substances and/or available reagents, the size of microparticles, and the level of resolution required for any method used for the array. The size of each address may be, for example, in the range of from 1-2 nm to several centimeters, though the address may have any size suited to an array.

The spatial arrangement and shape which define an address are designed so that the microarray is suited to a particular application. Addresses may be densely arranged or sparsely distributed, or subgrouped into a desired pattern appropriate for a particular type of material to be analyzed.

As used herein, the term "support" refers to a material which can carry cells, bacteria, viruses, polynucleotides, or polypeptides. Such a support may be made from any solid material which has a capability of binding to a biological molecule as used herein via covalent or noncovalent bond, or which may be induced to have such a capability.

Examples of materials used for supports include any material capable of forming a solid surface, such as, without limitation, glass, silica, silicon, ceramics, silicon dioxide, plastics, metals (including alloys), naturally-occurring and synthetic polymers (e.g., polystyrene, cellulose, chitosan, dextran, and nylon), and the like. Preferably, a support comprises a portion for producing hydrophobic bonds. A support may be formed of layers made of a plurality of materials. For example, a support may be made of an inorganic insulating material, such as glass, quartz glass, alumina, sapphire, forsterite, silicon carbide, silicon oxide, silicon nitride, or the like. A support may be made of an organic material, such as polyethylene, ethylene, polypropylene, polyisobutylene, polyethylene terephthalate, unsaturated polyester, fluorine-containing resin, polyvinyl chloride, polyvinylidene chloride, polyvinyl acetate, polyvinyl alcohol, polyvinyl acetal, acrylic resin, polyacrylonitrile, polystyrene, acetal resin, polycarbonate, polyamide, phenol resin, urea resin, epoxy resin, melamine resin, styrene-acrylonitrile copolymer, acrylonitrile-butadiene-styrene copolymer, silicone resin, polyphenylene oxide, polysulfone, and the like. Alternatively, nitrocellulose film, nylon film, PVDF film, or the like, which are used in blotting, may be used as a material for a support.

The varicella-zoster virus of the present invention can be used as an ingredient of a pharmaceutical composition for the treatment, prevention, and/or therapy of infectious diseases.

As used herein, the term "effective amount" in relation to a drug refers to an amount which causes the drug to exhibit intended efficacy. As used herein, an effective amount corresponding to a smallest concentration may be referred to as a minimum effective amount. Such a minimum effective amount is well known in the art. Typically, the minimum effective amount of a drug has been determined or can be determined as appropriate by those skilled in the art. The determination of such an effective amount can be achieved by actual administration, use of an animal model, or the like. The present invention is also useful for the determination of such an effective amount.

As used herein, the term "pharmaceutically acceptable carrier" refers to a material which is used for production of a pharmaceutical agent or an agricultural chemical (e.g., an animal drug), and has no adverse effect on effective ingredients. Examples of such a pharmaceutically acceptable carrier include, but are not limited to: antioxidants, preservatives, colorants, flavoring agents, diluents, emulsifiers, suspending agents, solvents, fillers, bulking agents, buffers, delivery vehicles, excipients, and/or agriculturalor pharmaceutical adjuvants.

The type and amount of a pharmaceutical agent used in the treatment method of the present invention can be easily determined by those skilled in the art based on information obtained by the method of the present invention (e.g., information relating to a disease) in view of the purpose of use, the target disease (type, severity, etc.), the subject's age, size, sex, and case history, the morphology and type of a site of a subject of administration, or the like. The frequency of subjecting a subject (patient) to the monitoring method of the present invention is also easily determined by those skilled in the art with respect to the purpose of use, the target disease (type, severity, etc.), the subject's age, size, sex, and case history, the progression of the therapy, and the like. Examples of the frequency of monitoring the state of a disease include once per day to once per several months (e.g., once per week to once per month). Preferably, monitoring is performed once per week to once per month with reference to the progression.

As used herein, the term "instructions" refers to a description of the method of the present invention for a person who performs administration, such as a medical doctor, a patient, or the like. Instructions state when to administer a medicament of the present invention, such as immediately after or before radiation therapy (e.g., within 24 hours, etc.). The instructions are prepared in accordance with a format defined by an authority of a country in which the present invention is practiced (e.g., Health, Labor and Welfare Ministry in Japan, Food and Drug Administration (FDA) in the U.S., and the like), explicitly describing that the instructions are approved by the authority. The instructions are so-called package insert and are typically provided in paper media. The instructions are not so limited and may be provided in the form of electronic media (e.g., web sites, electronic mails, and the like provided on the Internet).

In a therapy of the present invention, two or more pharmaceutical agents may be used as required. When two or more pharmaceutical agents are used, these agents may have similar properties or may be derived from similar origins, or alternatively, may have different properties or may be derived from different origins. A method of the present invention can be used to obtain information about the drug resistance level of a method of administering two or more pharmaceutical agents.

In the present invention, it will be appreciated by those skilled in the art that once the analysis result of a certain sugar chain structure has been correlated with a level of a disease concerning a similar type of organism, culture cell, tissue, animal (e.g., a mouse for a human) or the like, a corresponding sugar chain structure can be correlated with the disease level. Such matters are described and supported in, for example, "Doubut su Baiyosaibo Manual (Animal Culture Cell Manual), Seno et al. eds., Kyoritsu shuppan, 1993, the entirety of which is hereby incorporated by reference.

### (General techniques used herein)

Techniques used herein are within the technical scope of the present invention unless otherwise specified. These techniques are commonly used in the fields of sugar chain science, fluidics, micromachining, organic chemistry, biochemistry, genetic engineering, molecular biology, microbiology, genetics, and their relevant fields. The techniques are well described in documents described below and the documents mentioned herein elsewhere.

Micromachining is described in, for example, Campbell, S.A. (1996), The Science and Engineering of Microelectronic Fabrication, Oxford University Press; Zaut, P.V. (1996), Micromicroarray Fabrication: a Practical Guide to Semiconductor Processing, Semiconductor Services; Madou, M. J. (1997), Fundamentals of Microfabrication, CRC15 Press; Rai-Choudhury, P. (1997), Handbook of Microlithography, Micromachining & Microfabrication: Microlithography; and the like, the relevant portions of which are hereby incorporated by reference.

Molecular biology techniques, biochemistry techniques, and microbiology techniques used herein are well known and commonly used in the art, and are described in, for example, Maniatis, T. et al. (1989), Molecular Cloning: A Laboratory Manual, Cold Spring Harbor and its 3rd Ed. (2001); Ausubel, F.M. et al. eds, Current Protocols in Molecular Biology, John Wiley & Sons Inc., NY, 10158 (2000); Innis, M.A. (1990), PCR Protocols: A Guide to Methods and Applications, Academic Press; Innis, M.A. et al. (1995), PCR Strategies, Academic Press; Sninsky, J. J. et al. (1999), PCR Applications: Protocols for Functional Genomics, Academic Press; Gait, M.J. (1985), Oligonucleotide Synthesis: A Practical Approach, IRL Press; Gait, M. J. (1990), Oligonucleotide Synthesis: A Practical Approach, IRL Press; Eckstein, F. (1991), Oligonucleotides and Analogues: A Practical Approach, IRL Press; Adams, R.L. et al. (1992), The Biochemistry of the Nucleic Acids, Chapman & Hall; Shabarova, Z. et al. (1994), Advanced Organic Chemistry of Nucleic Acids, Weinheim; Blackburn, G.M. et al. (1996), Nucleic Acids in Chemistry and Biology, Oxford University Press; Hermanson, G.T. (1996), Bioconjugate Techniques, Academic Press; Method in Enzymology 230, 242, 247, Academic Press, 1994; Special issue, Jikken Igaku (Experimental Medicine) "Idenshi Donyu & Hatsugenkaiseki Jikkenho (Experimental Method for Gene introduction & Expression Analysis)", Yodo-sha, 1997; and the like. Relevant portions (or possibly the entirety) of each of these publications are herein incorporated by reference.

### (Description of preferred embodiments)

Hereinafter, the present invention will be described by way of embodiments. Embodiments described below are provided only for illustrative purposes. Accordingly, the scope of the present invention is not limited by the embodiments except as by the appended claims. It will be clearly appreciated by those skilled in the art that variations and modifications can be made without departing from the scope of the present invention with reference to the specification.

According to an aspect of the present invention, recombinant varicella-zoster virus is provided. Preferably, the varicella-zoster virus contains a BAC vector sequence in its genome sequence. By constructing a varicella-zoster virus genome containing a BAC vector sequence, it becomes possible to handle the varicella-zoster virus genome as the BAC molecule in bacteria. A BAC vector sequence used herein preferably contains an origin of replication derived from F plasmid, or alternatively may contain any origin of replication other than an origin of replication derived from F plasmid, as long as it has a sequence of 300 kb or more and can be held and grown as a bacterial artificial sequence in bacterial cells. The BAC vector of the present invention can be maintained and/or grow in bacterial host cells, preferably *E. coli* cells. Preferably, a portion of the BAC vector is inserted into a non-essential region of a varicella-zoster virus genome, so that it is possible to manipulate it as a BAC containing the varicella-zoster virus genome. When the BAC containing the varicella-zoster virus genome is introduced into a mammalian cell, the recombinant varicella-zoster virus can be produced and grown. As a host cell for the recombinant varicella-zoster virus, any mammalian cell which can grow a wild-type varicella-zoster virus strain can be used. Preferably, such a host cell is derived from a human, including, for example, but being not limited to, human MRC-5 cell, human HEL cell, and human WI-38.

### (Method for producing a BAC vector containing a human varicella-zoster virus genome)

Various techniques can be used to produce a BAC vector containing a human varicella-zoster virus by using a human varicella-zoster virus genome and a BAC vector.

An example of the technique using homologous recombination is a technique using a nucleic acid having a linear BAC vector sequence linked with a sequence homologous to a human varicella-zoster virus genome.

A method for producing a BAC vector comprising a human varicella-zoster virus genome by using a nucleic acid having a linear BAC vector sequence linked with a sequence homologous to a human varicella-zoster virus genome representatively comprises the steps of: (1) introducing the nucleic acid along with the human varicella-zoster virus genome into appropriate hosts (for example, into human established cell) ; (2) culturing the host cells to elicit homologous recombination between the homologous sequence linked with the linear BAC vector sequence and the human varicella-zoster virus genome sequence; (3) screening the host cells for one which contains the human varicella-zoster virus genome sequence having the BAC vector sequence incorporated due to the homologous recombination; (4) culturing the host cell and extracting a circular virus DNA.

Alternatively, in order to produce a BAC containing a human varicella-zoster virus genome using a human varicella-zoster virus genome and a BAC sequence, various methods, such as use of nucleic acid fragments obtained using restriction enzymes or the like, can be employed instead of homologous recombination.

A non-essential region of the varicella-zoster virus genome for introducing a BAC vector sequence thereinto selected from the group consisting of: the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.

Preferably non-essential regions are regions flanking gene 11 or regions flanking gene 12. This is because gene 11 and gene 12 are contiguous non-essential genes on the varicella-zoster virus genome, so that it is easy to design a nucleic acid for homologous recombination. Alternatively, a part of a BAC vector sequence may be inserted to the region in the ORF of gene 62 of varicella-zoster virus genome.

A BAC vector sequence used in the present invention preferably includes a recombinant protein-dependent recombinant sequence and/or a selectable marker. Preferably, the selectable marker sequence is a drug selectable marker and/or a gene encoding a green fluorescent protein. This is because the presence of a desired gene can be easily confirmed.

Varicella-zoster virus employed as a starting material in the present invention may be from wild strain or mutated strain. Preferably, an attenuated virus, for example, varicella-zoster virus having mutation in Oka vaccine strain or the gene 62 is used as varicella-zoster virus as a starting material. As an "attenuated varicella-zoster virus", a virus which comprises at least one of mutation of the gene 62, or more than one combination of mutation selected from the following group:
(a) base substitution at position 2110 for G;
(b) base substitution at position 3100 for G;
(c) base substitution at position 3818 for C;
(d) base substitution at position 4006 for G;
(e) base substitution at position 1251 for G;
(f) base substitution at position 2226 for G;
(g) base substitution at position 3657 for G;
(h) base substitution at position 162 for C;
(i) base substitution at position 225 for C;
(j) base substitution at position 523 for C;
(k) base substitution at position 1565 for C;
(1) base substitution at position 1763 for C;
(m) base substitution at position 2652 for C;
(n) base substitution at position 4052 for C; and
(o) base substitution at position 4193 for C,
   is included.

According to another aspect of the present invention, a vector used for production of the above-described virus and a method for producing the above-described virus are provided. According to still another aspect of the present invention, a pharmaceutical composition comprising the above-described virus and a pharmaceutical composition in the form of a vaccine are provided.

The recombinant human varicella-zoster virus of the present invention can be used as a vaccine, since it has many proteins which have the same structure as that of wild virus.

According to still another aspect of the present invention, a method for introducing mutation into a vector for producing a vaccine of the present invention is provided. The method comprises the steps of: introducing a vector into a bacterial host cell; introducing a plasmid vector containing a fragment consisting of a portion of a human varicella-zoster virus genome into the bacterial host cell, wherein the fragment has at least one mutation; culturing the bacterial host cell; and isolating a vector having a BAC vector sequence from the cultured bacterial host cell. In the above-described method, homologous recombination occurs between the vector for producing a vaccine of the present invention and the plasmid vector containing the fragment consisting of the portion of the human varicella-zoster virus genome, in bacterial host cells. As a result, the vector for producing the vaccine of the present invention has a mutation in the fragment consisting of the portion of the human varicella-zoster virus genome.

In the above-described method, the step of introducing the vector into bacterial host cells can be achieved by using various well-known methods, such as electroporation and the like. Similarly, the plasmid vector containing the fragment consisting of the portion of the human varicella-zoster virus genome can be introduced into bacterial host cells. As a technique for introducing a mutation into the fragment, a technique for introducing a mutation by using PCR is well known. For example, by using heat-resistant polymerase having no proofreading function, where one of the four nucleotides is in lower quantity, it is possible to introduce a mutation randomly. Alternatively, by PCR using a primer having a mutated base sequence, it is possible to introduce a desired mutation into a desired site. When the bacterial cell is cultured, homologous recombination occurs between the vector for producing the vaccine of the present invention and the plasmid vector containing the fragment consisting of the portion of the human varicella-zoster virus genome. As a result, the vector for producing the vaccine of the present invention has a mutation in the fragment consisting of the portion of the human varicella-zoster virus genome. In order to prepare a BAC vector sequence from a bacterial host cell, various well-known techniques (e.g., the alkaline method, etc.) and commercially available kits can be used.

According to another aspect of the present invention, another method for introducing a mutation into a vector for producing the vaccine of the present invention is provided. The method comprises the steps of: introducing the vector into a bacterial host cell; introducing a first plasmid vector containing a first fragment consisting of a portion of a human varicella-zoster virus genome into the bacterial host cell, wherein the first fragment has at least one mutation; introducing a second plasmid vector containing a second fragment consisting of a portion of the human varicella-zoster virus genome into the bacterial host cell, wherein the second fragment has at least one mutation and the second fragment is different from the first fragment; culturing the bacterial host cell; and isolating a vector having a BAC vector sequence from the cultured bacterial host cell.

According to an aspect of the present invention, a nucleic acid cassette which may be used for producing the vaccine of the present invention, is provided. The nucleic acid cassette preferably comprises a first fragment capable of homologous recombination with a human varicella-zoster virus genome in a host cell, a BAC vector sequence, and a second fragment capable of homologous recombination with a human varicella-zoster virus genome in the host cell, wherein the opposite ends of the BAC sequence are linked with the first fragment and second fragments, respectively. In this case, the first fragment and the second fragment are preferably at least 1 kb, at least 1.5 kb, or at least 2 kb in length. The first fragment and the second fragment preferably are at least 80% identity, at least 85% identity, at least 90% identity, or at least 95% identity to the human varicella-zoster virus genome sequence.

Preferably, the first and second fragments are independently derived from regions selected from the group consisting of the following regions of the varicella-zoster virus genome, or independently have at least 80%, 85%, 90%, or 95% identity to regions selected from the group consisting of the following regions of the varicella-zoster virus genome: the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 62, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, and the region flanking the ORF of gene 70.

Preferably, the first and second fragments are derived from different regions of the human varicella-zoster virus genome. The first and second fragments may be independently derived from a region flanking the ORF of gene 11 or 12. Preferably, the BAC vector sequence comprises a recombinant protein-dependent recombinant sequence and/or a selectable marker in order to control homologous recombination and easily detect a desired gene. The selectable marker may be either a drug selectable marker or a gene encoding a fluorescent protein (e.g., a green fluorescent protein, etc.). Representatively, the BAC vector sequence has a nucleic acid sequence set forth in SEQ ID NO.: 2, and the nucleic acid cassette has a nucleic acid sequence set forth in SEQ ID No.: 2.

### (Preparation of recombinant varicella-zoster virus containing a mutated gene)

A method of the present invention can be used to easily prepare a varicella-zoster virus having a varicella-zoster virus genome into which a mutated gene is introduced.

Such mutation introduction can be performed by using a method described below.

Into E. *coli,* (a) VZV-BAC-DNA plasmid and (b) a shuttle vector or a PCR product having a partial sequence of a varicella-zoster virus genome with any mutation as a mutated nucleic acid, are introduced. Homologous recombination is allowed to occur between VZV-BAC-DNA plasmid and the shuttle vector or PCR product, so that a foreign gene mutation can be introduced into VZV-BAC-DNA plasmid. Alternatively, a transposon can be used to randomly introduce a mutation. The VZV-BAC-DNA plasmid into which the mutation has been introduced, can be easily selected and grown in *E. coli.* By causing VZV-BAC-DNA having the mutation to produce a virus, the recombinant varicella-zoster virus can be obtained (Markus Wagner, TRENDS in Microbilogy, Vol. 10, No. 7, July 2002). Specific examples will be described below.
(1) Use of a temperature sensitive shuttle vector containing a mutated varicella-zoster virus as a mutated nucleic acid:
   Firstly, the shuttle vector and VZV-BAC-DNA plasmid are allowed to recombine via a first homologous region to generate a cointegrate in which the shuttle vector is linked with VZV-BAC-DNA plasmid. Next, since the replication origin of the shuttle vector is temperature-sensitive, the shuttle plasmid is removed. In a second recombination event, the cointegrated portion is removed. When the second recombination event occurs via the first homologous region, a plasmid having the same sequence as that of VZV-BAC-DNA used for the recombination is generated. In contrast, when the second recombination event occurs via a second homologous region different from the first homologous region, a modified VZV-BAC-DNA plasmid having the foreign gene contained in the shuttle vector is obtained. When the first homologous region and the second homologous region have substantially the same length, the probability that the second recombination event occurs in the second homologous region is substantially the same as the probability that the second recombination event occurs in the first homologous region. Therefore, about half of the resultant VZV-BAC-DNA plasmids are plasmids having the same sequence as that which has been used in the recombination, while about half thereof are plasmids having the foreign gene which has been introduced into the shuttle vector.
(2) Use of a linear DNA fragment:
   In this method, for example, by utilizing the recombination function of recET derived from prophage Rac or the recombination function of redαβ derived from bacteriophage λ, a linear DNA fragment is used to introduce a mutation into a circular VZV-BAC-DNA molecule. Specifically, a selectable marker flanking a target sequence and a linear DNA fragment containing a homologous sequence are introduced along with VZV-BAC-DNA into E. *coli* capable of homologous recombination. In order to avoid the degradation of the linear DNA within E. *coli,* it is preferable to use E. *coli* lacking exonuclease or cause expression of redγ (gam) which is an exonuclease inhibitor derived from a bacteriophage. The linear DNA has a region homologous to VZV-BAC-DNA plasmid on the opposite ends thereof. Homologous recombination occurs via the homologous region, thereby making it possible to introduce a desired sequence of the linear DNA fragment into VZV-BAC-DNA. RecET and redαβ exhibit homologous recombination via a homologous sequence having a length of about 25 to 50 nucleotides. Therefore, the recombination functions of recET and redαβ can be used more easily than recA-mediated homologous recombination.
(3) Use of a transposon:
   The function of a transposon element to insert into a nucleic acid in E. *coli* is used. For example, a transposon element containing a desired foreign gene and VZV-BAC-DNA are introduced into E. *coli* so that the transposon element is randomly inserted into VZV-BAC-DNA. Thereby, VZV-BAC-DNA having the inserted foreign gene is obtained.

Further, for example, it is also possible to introduce a random mutation in recombinant varicella-zoster virus genome by treating a host cell having recombinant varicella-zoster virus such as VZV-BAC-DNA employing a mutagenic agent (for example, nitrosoguanidine).

### (Formulation)

The present invention also provides methods of treatment and/or prevention of diseases or disorders (e.g., infectious diseases) by administration to a subject of an effective amount of a therapeutic/prophylactic agent. By the therapeutic/prophylactic agent is meant a composition of the present invention in combination with a pharmaceutically acceptable carrier type (e. g., a sterile carrier).

The therapeutic/prophylactic agent will be formulated and dosed in a fashion consistent with good medical practice, taking into account the clinical condition of the individual patient (especially the side effects of treatment with the therapeutic/prophylactic agent alone), the site of delivery, the method of administration, the scheduling of administration, and other factors known to those skilled in the art. The "effective amount" for purposes herein is thus determined by such considerations.

As a general proposition, the total pharmaceutically effective amount of the therapeutic/prophylactic agent administered parenterally per dose will be in the range of about 1 µg/kg/day to 10 mg/kg/day of patient body weight, although, as noted above, this will be subject to therapeutic discretion. More preferably, this dose is at least 0.01 mg/kg/day, and most preferably for humans between about 0.01 and 1 mg/kg/day for the cellular physiologically active material of the present invention. If given continuously, the therapeutic/prophylactic agent is typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect.

The therapeutic/prophylactic agents can be administered orally, rectally, parenterally, intracistemally, intravaginally, intraperitoneally, topically (as by powders, ointments, gels, drops or transdermal patch), or as an oral or nasal spray. "Pharmaceutically acceptable carrier" refers to a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

The therapeutic/prophylactic agents of the invention are also suitably administered by sustained-release systems. Suitable examples of sustained-release therapeutic/prophylactic agents are administered orally, rectally, parenterally, intracistemally, intravaginally, intraperitoneally, topically (as by powders, ointments, gels, drops or transdermal patch), or as an oral or nasal spray. "Pharmaceutically acceptable carrier" refers to a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

For parenteral administration, in one embodiment, the therapeutic/prophylactic agent is formulated generally by mixing it at the desired degree of purity, in a unit dosage injectable form (solution, suspension, or emulsion), with a pharmaceutically acceptable carrier, i. e., one that is non-toxic to recipients at the dosages and concentrations employed and is compatible with other ingredients of the formulation. For example, the formulation preferably does not include oxidizing agents and other compounds that are known to be deleterious to the therapeutic/prophylactic agent.

Generally, the formulations are prepared by contacting the therapeutic/prophylactic agent uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinyl pyrrolidone; amino acids,such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

Any pharmaceutical used for therapeutic administration can be free from organisms and viruses other than a virus as an effective ingredient, i.e., sterile. Sterility is readily accomplished by filtration through sterile filtration membranes (e.g., 0.2 micron membranes). Therapeutic/prophylactic agents generally are placed into a container having a sterile access port, for example, an intravenous solution bag or vial having a stopper pierceable by a hypodermic injection needle.

Therapeutic/prophylactic agents ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous therapeutic/prophylactic agent solution, and the resulting mixture is lyophilized. The infusion solution is prepared by reconstituting the lyophilized therapeutic/prophylactic agent using bacteriostatic Water-for-injection.

The invention also provides a pharmaceutical pack or kit comprising one or more containers filled with one or more of the ingredients of the therapeutic/prophylactic agents of the invention. Associated with such container (s) can be a notice in the form prescribed by a governmental agency regulating the manufacture, use or sale of pharmaceuticals or biological products, which notice reflects approval by the agency of manufacture, use or sale for human administration. In addition, the therapeutic/prophylactic agents may be employed in conjunction with other therapeutic compounds.

The therapeutic/prophylactic agents of the invention may be administered alone or in combination with other therapeutic agents. Therapeutic/prophylactic agents that may be administered in combination with the therapeutic/prophylactic agents of the invention, include but not limited to, chemotherapeutic agents, antibiotics, steroidal and nonsteroidal anti-inflammatories, conventional immunotherapeutic agents, cytokines and/or growth factors. Combinations may be administered either concomitantly, e.g., as an admixture, separately but simultaneously or concurrently; or sequentially. This includes presentations in which the combined agents are administered together as a therapeutic mixture, and also procedures in which the combined agents are administered separately but simultaneously, e.g., as through separate intravenous lines into the same individual. Administration "in combination" further includes the separate administration of one of the compounds or agents given first, followed by the second.

In certain embodiments, the therapeutic/prophylactic agents of the invention are administered in combination with antiretroviral agents, nucleoside reverse transcriptase inhibitors, nonnucleoside reverse transcriptase inhibitors, and/or protease inhibitors.

In a further embodiment, the therapeutic/prophylactic agents of the invention are administered in combination with an antibiotic agent. Antibiotic agents thatmaybe used include, but are not limited to, aminoglycoside antibiotics, polyene antibiotics, penicillin antibiotics, cephem antiboitics, peptide antibiotics, microride antibiotics, and tetracycline antibiotics.

In an additional embodiment, the therapeutic/prophylactic agents of the invention are administered alone or in combination with an anti-inflammatory agent. Anti-inflammatory agents that may be administered with the therapeutic/prophylactic agents of the invention include, but are not limited to, glucocorticoids and the nonsteroidal anti-inflammatories, aminoarylcarboxylic acid derivatives, arylacetic acid derivatives, arylbutyric acid derivatives, arylcarboxylic acids, arylpropionic acid derivatives, pyrazoles, pyrazolones, salicylic acid derivatives, thiazinecarboxamides, e-acetamidocaproic acid, S-adenosylmethionine, 3-amino-4-hydroxybutyric acid, amixetrine, bendazac, benzydamine, bucolome, difenpiramide, ditazol, emorfazone, guaiazulene, nabumetone, nimesulide, orgotein, oxaceprol, paranyline, perisoxal, pifoxime, proquazone, proxazole, and tenidap.

In a further embodiment, the therapeutic/prophylactic agent of the present invention is administered in combination with other therapeutic/prophylactic regimens (e.g., radiation therapy).

Hereinafter, the present invention will be described by way of examples. However, the present invention is not limited to these examples.

### (Example 1)

### (Preparation ofrecombinant varicella-zoster virus)

### (1: Preparation of BAC plasmid)

Plasmid PHA-2 used was kindly provided by Markus Wagner and Ulrich H. Koszinowski (Adler et al., (2000), J. Virol. 74: 6964-74). To prepare a recombinant virus, the region between gene 11 and gene 12 of varicella-zoster virus virus genome is selected as the insertion point of a BAC vector. This is because the insertion of a foreign nucleic acid into such a non-essential region was expected to have no adverse effect on the replication of varicella-zoster virus.

Fragments of the gene 11 ORF and the gene 12 ORF of varicella-zoster virus Oka strain was amplified with the genomic DNA of varicella-zoster virus Oka original strain as a template using primers VZ11F (SEQ ID NO. : 1) and VZ11R (SEQIDNO. : 2) and primers VZ12F (SEQIDNO.: 3) and VZ12R (SEQ ID NO.: 4), respectively.

### (2: Preparation of primers used for producing recombinant plasmid)

**[Table 1]**

| Primers used to produce a recombinant plasmid | | | |
|---|---|---|---|
| Primer | Sequence | Product (base pair) and plasmid | |
| VZ11F | 5-TATA ACTAGT GCGGCCGC TTACGAAAACGTGCATG-3' SpeI NotI | VZ ORF11(2652) | SK/VZ11-12 |
| VZ11R | 5'-CCCC ACCTGGT TTTATTTTACAAACTCCTTTGTGG-3' Sex AI | | |
| VZ12F | 5'-GCGC ACCAGGT CTCTGTTTAGACCTTAAAATTTG-3' SexAI | VZ ORF12(2164) | SK/VZ11-12 |
| VZ12R | 5'-TATA GCGGCCGC TTTTAATCTGGTTGTGGAAATG-3' NotI | | |

In the table, a restriction enzyme site in the sequence of an oligonucleotide is underlined, while an italicized sequence indicates a base which does not exist in the VZV sequence.

Fragments of the gene 11 ORF and the gene 12 ORF, which were produced by PCR were digested by SpeI/SexAI and NotI/SexAI, respectively. Two PCR fragments were cloned to pBluescript SK- (Stratagene) digested by SpeI and NotI. The resultant plasmid was designated as SK/VZ11-12.

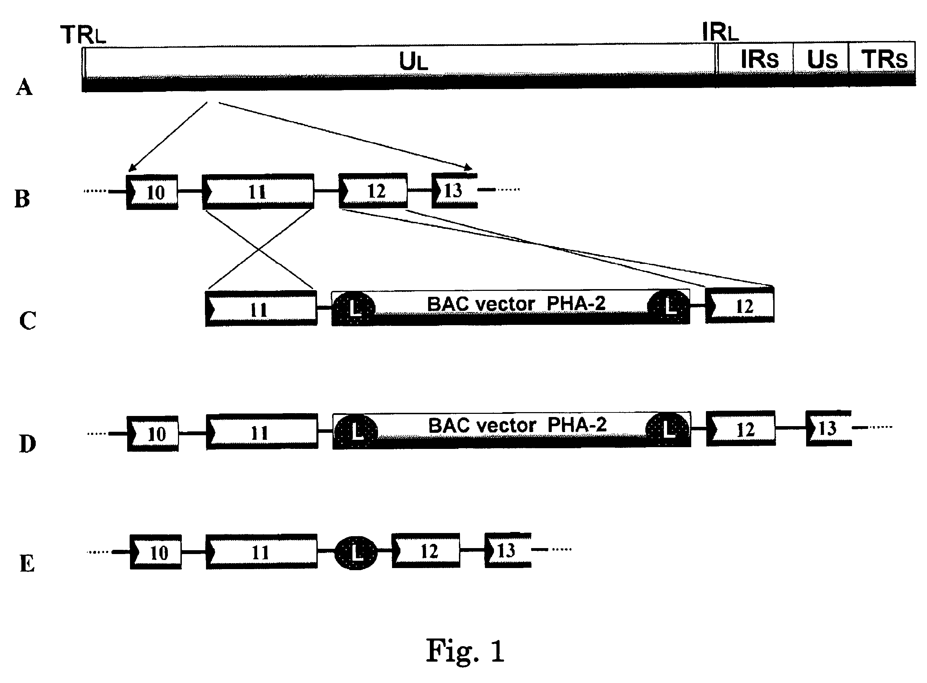

Plasmid pHA-2 was digested by Pacl, and then, this site was blunt-ended by treatment with T4 DNA polymerase. This plasmid was cloned into the SexAI site which was blunt-ended in SK/VZ11-12. The resulting plasmid was designated as pHA-2/VZ11-12 (Figure 1C).

As shown in Figure 1, VZV genome (A) is about 125 kbp in length, and comprises terminal repeat (TR) DNA domain, uniquely long (UR) DNA domain, internal repeat (IR) DNA domain, and uniquely short (US) DNA domain. To construct a recombinant plasmid PHA-2/VZV11-12(C), as mentioned above, an ORF 11 fragment and an ORF 12 fragment of the VZV genome were produced by PCR amplification using appropriate primers. This recombinant plasmid pHA-2/VZ11-12 included flanking homologous sequence of about 2.0 kbp, which is adjacent to loxP site (L), and the BAC vector.

### (3: Preparation of recombinant virus by homologous recombination)

The prepared plasmid pHA-2/VZ11-12 contains a guanine phosphoribosyl transferase (gpt) gene as a selectable marker. The BAC vector sequence is sandwiched between two loxP sequences. Therefore, the BAC vector sequence sandwiched between the loxP sequences can be efficiently removed by the action of Cre recombinase. In addition, cells into which the plasmid containing the BAC vector sequence has been introduced can be easily confirmed by the fluorescence of a green fluorescent protein (GFP).

The plasmid was digested with NotI for linearization. Nucleofector unit (Amaxa) was used to transfect HEL cells cultured to confluent in a 75-cm² plastic flask with 0.2 µg of the linearized pHA-2/VZ11-12by electroporation. One day after transfection, the transfected cells were infected with varicella-zoster virus Oka strain.

Mycophenolic acid (50 µM) and 200 µM xanthine were used to screen recombinant viruses based on the gpt gene. In the HEL cells, the cytopathic effect (CPE) typical for varicella-zoster virus was observed. Some of the cells could be confirmed under a microscope to express the GFP. This result indicates that the BAC vector was inserted into the varicella-zoster virus genome and the GFP gene was expressed.

### (4: Enrichment of recombinant virus and introduction of it into E. coli)

A recombinant virus was enriched by selection using the gpt gene in combination with mycophenolic acid and xanthine, and use of limiting dilution assay of 96 well plate. From the infected cells, circular virus DNA was extracted by Hirt's method (Hirt, (1967), J. M. Biol, 26: 365-9). The extracted DNA was introduced into *E. coli* DH10B by the electroporation method (0.2-cm cuvette, 2.5 kV) using the gene pulser (Bio-Rad) for transformation. *E. coli* containing VZV-BAC-DNA was obtained by screening on agar containing 17 µg/ml chloramphenicol.

### (5: Stability of VZV-BAC-DNA plasmid in E. coli)

*E. coli* including the BAC vector (VZV-BAC-DNA) including varicella-zoster virus genome was cultured for 22-24 hours in LB Broth, passaged 3 times by employing the same method, and finally, selected on an agar-plate including chloramphenicol. Five clones were picked from the passaged E. *coli,* and each of the five clones were cultured in large scale in LB Broth by employing the same method, and DNA was extracted. VZV-BAC-DNA was extracted from E.coli using the Nucleobond PC 100 kit (Macherey-Nagel) in accordance with the protocol accompanying the kit. The resultant five clones and the original VZV-BAC-DNA were digested with restriction enzyme BamHI. The restriction enzyme patterns were verified on an agarose gel by electrophoresis (the result is not shown) . All five clones were compared with the original VZV BAC plasmid. As a result, the identical restriction enzyme pattern was shown on the agarose gel. This indicated high stability of VZV plasmid in E. *coli.*

In these Figures, the original VZV-BAC-DNA and the VZV-BAC-DNA, which was passaged 3 times showed the identical electrophoratic pattern. This indicates that VZV-BAC-DNA plasmid is stable in E. *coli.*

### (6: Production of virus from VZV-BAC-DNA)

BAC cloned VZV rV01 (Figure 1D) was produced in HEL cells by homologous recombination of a recombinant plasmid PHA-2/VZV11-12 and VZV virus. Specifically, HEL cells cultured to confluent in a 75-cm² plastic flask were transfected with 1 µg VZV-BAC-DNA using Nucleofector unit (Amaxa), and then the HEL cells were cultured to confluency in a 75-cm² plastic flask and were passaged two days after transfection. Two or three days after passage, typical CPE of varicella-zoster was observed. Also, cells, in which CPE were observed, were confirmed under a fluorescent microscope to express the GFP gene. Consequently, it was confirmed that the recombinant varicella-zoster virus could be produced using VZV-BAC-DNA. The produced recombinant varicella-zoster virus was designated as rV01 (Figure 1D). VZV BAC plasmid was produced by introducing circular BAC cloned genome in E. *coli.*

### (7: Cutting out of BAC vector sequence)

Recombinant adenovirus (AxCANCre) capable of expressing Cre recombinase (Kanegae et al., (1995) Nucleic Acids Res 23:3816-21) was kindly provided by Yasushi Kawaguchi of the Nagoya University. VZV rV02 (Figure 1E; L represents loxP site) was produced by superinfection with BAC cloned VZV rV01 and the recombinant adenovirus (AxCANCre) using this recombinant adenovirus. Specifically, HEL cells were infected with the recombinant adenovirus at a MOI (multiplicity of infection) of 100. After 2 hours of virus adsorption, the cells were washed with PBS(-), followed by culturing in DMEM medium containing 5% FCS. The HEL cells were superinfected with recombinant varicella-zoster virus rV01 24 hours after infection with recombinant adenovirus. A control experiment was conducted to confirm that the recombinant adenovirus expressed Cre recombinase and that a BAC vector sequence was efficiently cut out from rV01 genome. The resultant varicella virus was designated rV02 (Figure 1, E). The result of DNA sequencing confirmed that rV02 was a nucleic acid produced by cutting out a BAC vector sequence from rV01.

DNA which is extracted from cells infected with varicella-zoster virus Oka strain and VZV-BAC-DNA derived from E. *coli* were digested by restrection enzyme BamHI. Fragments derived from recombinant varicella-zoster virus rV02 DNA are larger than those of varicella-zoster virus Oka strain DNA due to the remaining one loxP sequence.

Regarding the electrophoratic pattern of VZV-BAC-DNA compared with parent strain, due to insertion of BAC vector, BamHI fragments of about 8.1 kbp disappeared, while BamHI fragments of about 7.8 kbp and 9. 2 kbp were added. Also,a BamHI fragment of about 8.2 kbp of DNA, which was extracted from recombinant varicella virus rV02 infected cells, increased in size in comparison with the about 8.1 kbp BamHI fragment of the parent varicella-zoster virus, because of one loxP sequence which remains when BAC vector sequence was cut out.

### (Example 2)

### (Characterization of recombinant varicella-zoster virus)

### (1: Comparison of growth ability of recombinant viruses)

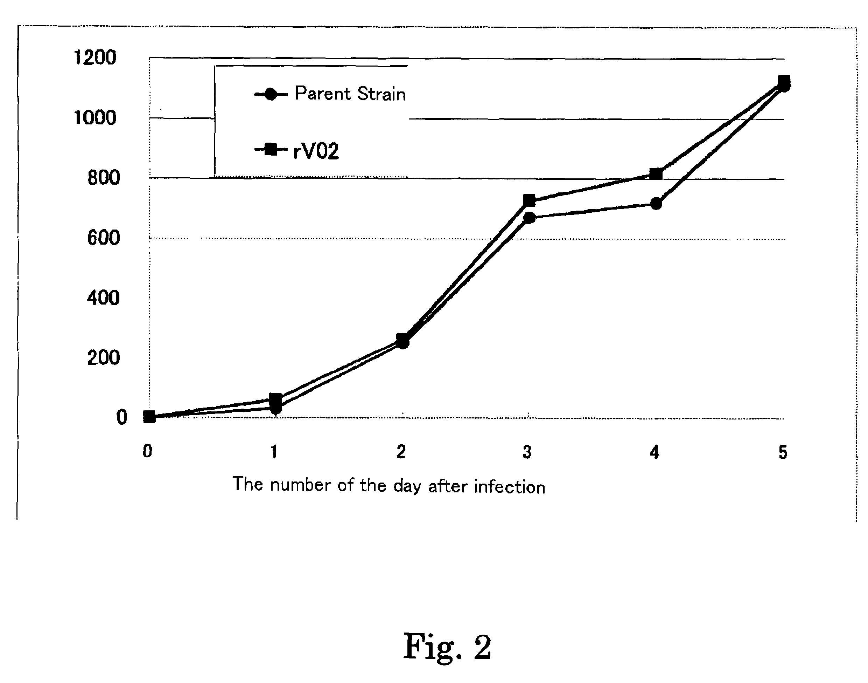

Varicella-zoster virus Oka strain and the obtained recombinant varicella-zoster virusrV02 are compared in terms of the growth ability in HEL cells using the infectious center assay method (Gomi et al., (2002) J. Virol 76 :11447-59). HEL cells in a 35mmdish were infected at a MOI of 0.01 PFU/cell, and the infected cells were washed. After varicella-zoster virus Oka strain and rV02 strain with which HEL cells were infected were cultured from day 0 to day 5, and harvested with trypsin, these strains were infected to new HEL cells to compare the replication ability thereof. The numbers of the infected cells are normalized to the initial virus titer/dish. Multiple growth indicates the number of infected cells transmitted from one infected cell at 0 day. The result is shown at Figure 2. As shown in Figure 2, it indicates that the obtained recombinant varicella virus rV02 exhibits the same replication ability as that of varicella virus Oka strain (parent strain) *in vitro.*

### (Example 3)

### (Production of mutant recombinant varicella-zoster virus with low pathogenicity)

According to the present invention, it is possible to prepare a mutant recombinant varicella-zoster virus and to obtain a mutant varicella-zoster virus strain with low pathogenicity in a mutated virus using the following method.

### (1: Preparation of mutant recombinantvaricella-zoster virus)

As a method for preparing mutant recombinant varicella-zoster virus including, for example, homologous recombination between a nucleic acid containing a mutated gene and VZV-BAC-DNA plasmid to produce mutant recombinant varicella-zoster virus. A mutated gene, which is used to cause homologous recombination with VZV-BAC-DNA plasmid may include random mutation and may include site-directed mutation. By employing each of the above methods, it is possible to obtain a population of mutant recombinant varicella-zoster virus with random mutation and a population of mutant recombinant varicella-zoster virus with site-directed mutation. The detailed description of the foregoing is as follows.

### (1.1: Preparation of mutant recombinant varicella-zoster virus with random mutation)

It is known that some of viruses which contain mutation in gene 62 of varicella-zoster virus genome are attenuated viruses. Therefore, in the present Example, gene 62 to which a mutation is randomly introduced using PCR is produced. The method of mutagenesis using PCR is well known. For example, it is possible to introduce a mutation randomly by using thermostable polymerase without proofreading function under the condition that the amount of one of the four nucleotides is small. Optionally, a marker gene such as a drug-resistance gene may be linked to the mutated 62 gene.

Thus, the prepared mutated gene 62 is introduced into E. *coli* with VZV-BAC-DNA plasmid according to Example 1 (4: Enrichment of recombinant virus and introduction of it into *E. coli) ,* and then, homologous recombination was allowed to occur between mutated gene 62 and VZV-BAC-DNA. After that, according to the method described in Example 1, DNA of varicella-zoster virus which causes homologous recombination is isolated and introduced to E. *coli,* and E. *coli* including VZV-BAC-DNA which causes homologous recombination is obtained.

The obtained plurality of E. *coli* contains VZV-BAC-DNA including gene 62 having mutations which are different from each other. Then, the degree of pathogenicity of varicella-zoster virus which is produced by mutant VZV-BAC-DNA included in each E. *coli* is screened using a method below (2: method of examining the pathogenicity of varicella-zoster virus).

### (1.2: Preparation of mutant recombinant varicella-zoster virus containing a site-directed mutation)

The methods for introducing the desired site-directed mutation is well-known in the art. For example, the full-length gene containing the desired mutation is prepared by conducting PCR using primers containing the desired mutation so as to prepare the fragment of the gene containing the desired mutation, and then, by further conducting PCR using the fragments of the mutated gene or by treating with an enzyme, such as a restriction enzyme.

Thus, mutant recombinant varicella-zoster virus containing a site-directed mutation is prepared using the procedure of above-mentioned (1.1.), regarding the prepared mutated gene.

### (2:method ofexamining the pathogenicity of varicella-zoster virus)

The two methods for examining the pathogenicity of varicella-zoster virus have been established.

As a method using an animal model, the method for evaluating the pathogenicity by producing a severe combined immunodeficient (SCID) mouse to which human skin is transplanted, and then, to infect the mouse with varicella-zoster virus is well-known (J. Virol. 1998 Feb; 72(2): 965-74,).

On the other hand, as a method for evaluating the pathogenicity in vitro is well-known, which comprises: placing monolayer cultured human melanoma in a lower-well of a two-layered well, which are separated by a trans-well of pore size 3 µm; placing cord-blood mononuclear cells (CBMC) infected with varicella-zoster virus in the upper-well; and culturing the cells for 7-8 days; then observing CPE (cytopathic effect) of the melanoma (J. Virol. 2000 Feb; 74(4): 1864-70).

Although it is not the method for confirming the pathogenicity directly, according to the previous study of the present inventors (J. Virol. 2002 Nov; 76 (22) : 11447-59), close relationships between the pathogenicity and the proliferation of a virus is understood, thus, it is also possible to evaluate the pathogenicity indirectly by examining the proliferation of cell-to-cell employing infectious center assay.

### (Example 4)

### (Production of vaccine)

The recombinant varicella-zoster virus obtained in Example 1 is inoculated in MRC-5 cell culture in 20 Roux bottles having a culture area of 210 cm², followed by culturing. After completion of culturing, culture medium is discarded, and the infected cells in each Roux bottle are washed with 200 ml of PBS (-) twice. Next, 20 ml of 0.03% (w/v) EDTA-3Na is overlaid on the infected cells in each Roux bottle, so that the cells are detached from the wall of the Roux bottle and suspended. The infected cell suspension in each Roux bottle is pooled, followed by centrifugation at 2,000 rpm for 10 minutes at 4°C to collect a pellet of the infected cells. The cells are resuspended in 100 ml of PBS (-), followed by freezing and thawing once. Next, the cells are subjected to ultrasonication in ice bath (20 KHz, 150 mA, 0.3 sec/ml), followed by centrifugation at 3,000 rpm for 20 minutes at 4°C. The supernatant containing viruses released from the cells is collected, which is used as a live vaccine stock solution. 30 ml of the stock solution is sampled for examination, while saccharose and gelatin hydolysate dissolved in PBS(-), which serves as a vaccine stabilizer, is added and mixed into the remaining stock solution (70 ml) to a final concentration of 5% (w/v) and 2.5% (w/v). As a result, 140 ml of a live vaccine final bulk was prepared. 30 ml of the final bulk is sampled for examination. Thereafter, the remainder of the bulk is dispensed into 3 ml volume vials (0.5 ml for each). After lyophilization, the vial is filled with nitrogen gas and is closed with a rubber cap to hermetically seal the inside of the vial. The live vaccine aliquots are preserved at 4°C. Immediately before use, 0.5 ml of distilled water for injection is added to the lyophilized contents which are completely dissolved. On the other hand, the above-described sampled vaccine stock solution and final bulk, and 20 aliquots are subjected to assay tests. The tests are conducted to confirm safety, effectiveness, and uniformity for qualification of a live vaccine, taking into consideration the Guidelines for Biological Formulations defined under Notice No. 195 of Ministry of Health and Welfare (1989) and the guideline "recombinant precipitation hepatitis B vaccine (derived from yeast) " as defined therein. The results of the tests show that the above-described aliquot has a virus content of 2×10⁴ PFU (plaque-formingunit)/0.5 ml. When passing each test described in the guidelines, the vaccine is subsequently used as a qualified live vaccine.

### (Example 5)

### (Determination of immunogenicity of recombinant varicella-zoster virus vaccine)

Immunogenicity of recombinant varicella-zoster virus vaccine strain produced in Example 4 is measured using guinea-pigs. Oka strain live vaccine is used as a control. These vaccines are subcutaneously vaccined to each of three guinea pigs of 3 weeks old (average weight is 250g). Vaccination is adjusted by diluting each vaccine using PBS (-) so that the amount of recombinant strain and Oka strain live vaccine is 3,000 PFU/guinea pig or 2,000 PFU/guinea pig. Four, six, and eight weeks after vaccination, blood is collected from the vein in the femoral region of each vaccined guinea pig to measure the antibody value in the blood. The Neutralizing test method (Journal of General Virology, 61, 255-269, 1982) is employed for measurement of antibody value. It is confirmed that recombinant varicella-zoster virus vaccine elicit anti-VZV antibody to the same degree with Oka strain. From these results, recombinant varicella-zoster virus vaccine with good immunogenicity is selected.

Although certain preferred embodiments have been described herein, it is not intended that such embodiments be construed as limitations on the scope of the invention except as set forth in the appended claims. Various other modifications and equivalents will be apparent to and can be readily made by those skilled in the art, after reading the description herein, without departing from the scope and spirit of this invention. All patents, published patent applications and publications cited herein are incorporated by reference as if set forth fully herein.

The present invention provides a method for producing recombinant varicella-zoster virus from a single virus strain using, for example, BAC (*E. coli* artificial chromosome), and recombinant varicella-zoster virus produced by the method. The present invention also provides a pharmaceutical composition comprising recombinant varicella-zoster virus.

Further, the present invention provides a vector comprising a varicella-zoster virus genomic gene and a BAC vector sequence, a cell containing such a vector, and a nucleic acid cassette comprising a fragment capable of homologous recombination with a varicella-zoster virus genome, and a BAC vector sequence.

### (Description of sequence table)

SEQ ID NO.: 1, VZ11F primer
SEQ ID NO.: 2, VZ11R primer
SEQ ID NO.: 3, VZ12F primer
SEQ ID NO.: 4, VZ12R primer
SEQ ID NO.: 5, sequence of the gene 62
SEQ ID NO.: 6, sequence of the gene 62
SEQ ID NO.: 7, sequence of plasmid PHA-2
SEQ ID NO.: 8, varicella-zoster virus Dumas strain
SEQ ID NO.: 9, 5'→3' direction 1134 to 1850 of SEQ IDNO.: 8, nucleic acid sequence encoded (gene 2)
SEQ ID NO.: 10,5'→3' direction 8607 to 9386 of SEQ ID NO.: 8, nucleic acid sequence encoded (gene 7)
SEQ ID NO.: 11, 5'→3' direction 10642 to 10902 of SEQ ID NO.: 8, nucleic acid sequence encoded (gene 9A)
SEQ ID NO.: 12, 5'→3' direction 11009 to 11917 of SEQ ID NO.: 8, nucleic acid sequence encoded (gene 9)
SEQ ID NO.: 13, 5'→3' direction 12160 to 13392 of SEQ ID NO.: 8, nucleic acid sequence encoded (gene 10)
SEQ ID NO.: 14, 5'→3' direction 13590 to 16049 of SEQ ID NO.: 8, nucleic acid sequence encoded (gene 11)
SEQ ID NO.: 15, 5'→3' direction 16214 to 18199 of SEQ ID NO.: 8, nucleic acid sequence encoded (gene 12)
SEQ ID NO.: 16, 5'→3' direction 18441 to 19346 of SEQ ID NO.: 8, nucleic acid sequence encoded (gene 13)
SEQ ID NO.: 17, 5'→3' direction 24149 to 25516 of SEQ ID NO.: 8, nucleic acid sequence encoded (gene 17)
SEQ ID NO.: 18, 5'→3' direction 30759 to 33875 of SEQ ID NO.: 8, nucleic acid sequence encoded (gene 21)
SEQ ID NO.: 19, 5'→3' direction 34083 to 42374 of SEQ ID NO.: 8, nucleic acid sequence encoded (gene 22)
SEQ ID NO.: 20, 5'→3' direction 44506 to 46263 of SEQ ID NO.: 8, nucleic acid sequence encoded (gene 26)
SEQ ID NO.: 21, 5'→3' direction 50857 to 54471 of SEQ ID NO.: 8, nucleic acid sequence encoded (gene 29)
SEQ ID NO.: 22, 5'→3' direction 54651 to 56963 of SEQ ID NO.: 8, nucleic acid sequence encoded (gene 30)
SEQ ID NO.: 23, 5'→3' direction 57008 to 59614 of SEQ ID NO.: 8, nucleic acid sequence encoded (gene 31)
SEQ ID NO.: 24, 5'→3' direction 59766 to 60197 of SEQ ID NO.: 8, nucleic acid sequence encoded (gene 32)
SEQ ID NO.: 25, 5'→3' direction 64807 to 65832 of SEQ ID NO.: 8, nucleic acid sequence encoded (gene 36)
SEQ ID NO.: 26, 5'→3' direction 66074 to 68599 of SEQ ID NO.: 8, nucleic acid sequence encoded (gene 37)
SEQ ID NO.: 27, 5'→3' direction 70633 to 71355 of SEQ ID NO.: 8, nucleic acid sequence encoded (gene 39)
SEQ ID NO.: 28, 5'→3' direction 71540 to 75730 of SEQ ID NO.: 8, nucleic acid sequence encoded (gene 40)
SEQ ID NO.: 29, 5'→3' direction 75847 to 76797 of SEQ ID NO.: 8, nucleic acid sequence encoded (gene 41)
SEQ ID NO.: 30, 5'→3' direction 78170 to 80200 of SEQ ID NO.: 8, nucleic acid sequence encoded (gene 43)
SEQ ID NO.: 31, 5'→3' direction 80360 to 81451 of SEQ ID NO.: 8, nucleic acid sequence encoded (gene 44)
SEQ ID NO.: 32, 5'→3' direction 82719 to 83318 of SEQ ID NO.: 8, nucleic acid sequence encoded (gene 46)
SEQ ID NO.: 33, 5'→3' direction 84667 to 86322 of SEQ ID NO.: 8, nucleic acid sequence encoded (gene 48)
SEQ ID NO.: 34, 5'→3' direction 87881 to 90388 of SEQ ID NO.: 8, nucleic acid sequence encoded (gene 51)
SEQ ID NO.: 35, 5'→3' direction 90493 to 92808 of SEQ ID NO.: 8, ucleic acid sequence encoded (gene 52)
SEQ ID NO.: 36, 5'→3' direction 95996 to 98641 of SEQ ID NO.: 8, nucleic acid sequence encoded (gene 55)
SEQ ID NO.: 37, 5'→3' direction 110581 to 111417 of SEQ ID NO.: 8, nucleic acid sequence encoded (gene 63)
SEQ ID NO.: 38, 5'→3' direction 111565 to 112107 of SEQ ID NO.: 8, nucleic acid sequence encoded (gene 64)
SEQ ID NO.: 39, 5'→3' direction 113037 to 114218 of SEQ ID NO.: 8, nucleic acid sequence encoded (gene 66)
SEQ ID NO. :40, 5'→3' direction 114496 to 115560 of SEQ ID NO.: 8, nucleic acid sequence encoded (gene 67)
SEQ ID NO.: 41, 5'→3' direction 115808 to 117679 of SEQ ID NO.: 8, nucleic acid sequence encoded (gene 68)
SEQ ID NO.: 42, 5'→3' direction 120764 to 124696 of SEQ ID NO.: 8, nucleic acid sequence encoded (gene 71)
SEQ ID NO.: 43 partial sequence of SEQ ID No.:8 (gene 27) SEQ ID NO.: 44, 5'→3' direction 1 to 999 of SEQ ID NO. : 43, nucleic acid sequence encoded (gene 27)
SEQ ID NO.: 45, partial sequence of SEQ ID No.:8 (gene 47) SEQ ID NO.: 46, 5'→3' direction 1 to 1530 of SEQ ID NO.: 45, nucleic acid sequence encoded (gene 47)
SEQ ID NO.: 47, partial sequence of SEQ ID No.:8
SEQ ID NO.: 48, 5'→3' direction 1 to 243 of SEQ ID NO.: 47, nucleic acid sequence encoded (gene 49)
SEQ ID NO.: 49, partial sequence of SEQ ID No.:8
SEQ ID NO.: 50, 5'→3' direction 1 to 732 of SEQ ID NO.: 49, nucleic acid sequence encoded (gene 56)
SEQ ID NO.: 51, partial sequence of SEQ ID No.:8
SEQ ID NO.: 52, 3'→5' direction 118480 to 119316 of SEQ ID NO.: 8, nucleic acid sequence encoded (corresponding to 5569 to 6405 of SEQ ID No.: 51)(gene 70)
SEQ ID NO.: 53, 3'→5' direction 117790 to 118332 of SEQ ID NO.: 8, nucleic acid sequence encoded (corresponding to 6553 to 7095 of SEQ ID No.: 51)(gene 69)
SEQ ID NO.: 54, 3'→5' direction 112332 to 112640 of SEQ ID NO.: 8, nucleic acid sequence encoded (corresponding to 12245 to 12553 of SEQ ID No.: 51)(gene 65)
SEQ ID NO.: 55, 3'→5' direction 105201 to 109133 of SEQ ID NO.: 8, nucleic acid sequence encoded (corresponding to 15752 to 19684 of SEQ ID No.: 51)(gene 62)
SEQ ID NO.: 56, 3'→5' direction 103082 to 104485 of SEQ ID NO.: 8, nucleic acid sequence encoded (corresponding to 20400 to 21803 of SEQ ID No.: 51)(gene 61)
SEQ ID NO.: 57, 3'→5' direction 100302 to 101219 of SEQ ID NO.: 8, nucleic acid sequence encoded (corresponding to 23666 to 24583 of SEQ ID No.: 51)(gene 59)
SEQ ID NO.: 58, 3'→5' direction 99411 to 99626 of SEQ ID NO.: 8, nucleic acid sequence encoded (corresponding to 25259 to 25474 of SEQ ID No.: 51)(gene 57)
SEQ ID NO.: 59, 3'→5' direction 92855 to 93850 of SEQ ID NO.: 8, nucleic acid sequence encoded (corresponding to 31035 to 32030 of SEQ ID No.: 51)(gene 53)
SEQ ID NO.: 60, 3'→5' direction 68668 to 70293 of SEQ ID NO.: 8, nucleic acid sequence encoded (corresponding to 54592 to 56217 of SEQ ID No.: 51)(gene 38)
SEQ ID NO.: 61, 3'→5' direction 63977 to 64753 of SEQ ID NO.: 8, nucleic acid sequence encoded (corresponding to 60132 to 60908 of SEQ ID No.: 51)(gene 35)
SEQ ID NO.: 62, 3'→5' direction 62171 to 63910 of SEQ ID NO.: 8, nucleic acid sequence encoded (corresponding to 60975 to 62714 of SEQ ID No.: 51)(gene 34)
SEQ ID NO.: 63, 3'→5' direction 60321 to 62138 of SEQ ID NO.: 8, nucleic acid sequence encoded (corresponding to 62747 to 64564 of SEQ ID No.: 51)(gene 33)
SEQ ID NO.: 64, 3'→5' direction 47052 to 50636 of SEQ ID NO.: 8, nucleic acid sequence encoded (corresponding to 74249 to 77833 of SEQ ID No.: 51)(gene 28)
SEQ ID NO.: 65, 3'→5' direction 44148 to 44618 of SEQ ID NO.: 8, nucleic acid sequence encoded (corresponding to 80267 to 80737 of SEQ ID No.: 51)(gene 25)
SEQ ID NO.: 66, 3'→5' direction 43212 to 44021 of SEQ ID NO.: 8, nucleic acid sequence encoded (corresponding to 80864 to 81673 of SEQ ID No.: 51)(gene 24)
SEQ ID NO.: 67, 3'→5' direction 42431 to 43138 of SEQ ID NO.: 8, nucleic acid sequence encoded (corresponding to 81747 to 82454 of SEQ ID No.: 51)(gene 23)
SEQ ID NO.: 68, 3'→5' direction 29024 to 30475 of SEQ ID NO.: 8, nucleic acid sequence encoded (corresponding to 94410 to 95861 of SEQ ID No.: 51)(gene 20)
SEQ ID NO.: 69, 3'→5' direction 26518 to 28845 of SEQ ID NO.: 8, nucleic acid sequence encoded (corresponding to 96040 to 98392 of SEQ ID No.: 51)(gene 19)
SEQ ID NO.: 70, 3'→5' direction 25573 to 26493 of SEQ ID NO.: 8, nucleic acid sequence encoded (corresponding to 98392 to 99312 of SEQ ID No.: 51)(gene 18)
SEQ ID NO.: 71, 3'→5' direction 22568 to 23794 of SEQ ID NO.: 8, nucleic acid sequence encoded (corresponding to 101091 to 102317 of SEQ ID No.: 51)(gene 16)
SEQ ID NO.: 72, 3'→5' direction 21258 to 22478 of SEQ ID NO.: 8, nucleic acid sequence encoded (corresponding to 102407 to 103627 of SEQ ID No.: 51)(gene 15)
SEQ ID NO.: 73, 3'→5' direction 19431 to 21113 of SEQ ID NO.: 8, nucleic acid sequence encoded (corresponding to 103772 to 105454 of SEQ ID No.: 51)(gene 14)
SEQ ID NO.: 74, 3'→5' direction 9477 to 10667 of SEQ ID NO.: 8, nucleic acid sequence encoded (corresponding to 114218 to 115408 of SEQ ID No.: 51)(gene 8)
SEQ ID NO.: 75,3'→5' direction 5326 to 8577 of SEQ IDNO.: 8, nucleic acid sequence encoded (corresponding to 116308 to 119559 of SEQ ID No.: 51)(gene 6)
SEQ ID NO.: 76,3'→5' direction 4252 to 5274 of SEQ IDNO.: 8, nucleic acid sequence encoded (corresponding to 119611 to 120633 of SEQ ID No.: 51)(gene 5)
SEQ ID NO.: 77,3'→5' direction 2783 to 4141 of SEQ IDNO. : 8, nucleic acid sequence encoded (corresponding to 120744 to 122102 of SEQ ID No.: 51)(gene 4)
SEQ ID NO.: 78,3'→5' direction 1908 to 2447 of SEQ IDNO.: 8, nucleic acid sequence encoded (corresponding to 122438 to 122977 of SEQ ID No.: 51)(gene 3)
SEQ ID NO.: 79, 3'→5' direction 589 to 915 of SEQ ID NO.: 8, nucleic acid sequence encoded (corresponding to 123970 to 124296 of SEQ ID No.: 51)(gene 1)
SEQ ID NO.: 80, partial sequence of SEQ ID No.:51
SEQ ID NO.: 81, 3'→5' direction 1 to 1056 and 4556 to 5740 of SEQ ID NO.: 80, nucleic acid sequence encoded (corresponding to 46847 to 48034 and 42292 to 43347 of SEQ ID No.: 51)(gene 42 and gene 45)
SEQ ID NO.: 82, partial sequence of SEQ ID No.:51
SEQ ID NO.: 83, 3'→5' direction 1 to 1305 of SEQ ID NO.: 82, nucleic acid sequence encoded (corresponding to 123580 to 124884 of SEQ ID No.: 51)(gene 50)
SEQ ID NO.: 84, partial sequence of SEQ ID No.:51
SEQ ID NO.: 85, 3'→5' direction 1 to 2307 of SEQ ID NO. : 84, nucleic acid sequence encoded (corresponding to 122578 to 124884 of SEQ ID No.: 51)(gene 54)
SEQ ID NO.: 86, partial sequence of SEQ ID No.:51
SEQ ID NO.: 87, 3'→5' direction 1 to 663 of SEQ ID NO.: 86, nucleic acid sequence encoded (corresponding to 124222 to 124884 of SEQ ID No.: 51)(gene 58)
SEQ ID NO.: 88, partial sequence of SEQ ID No.:51
SEQ ID NO.: 89, 3'→5' direction 1 to 427 of SEQ ID NO.: 88, nucleic acid sequence encoded (corresponding to 124458 to 124884 of SEQ ID No.: 51)(gene 60)
SEQ ID NO.: 90, partial sequence of SEQ ID No.:51
SEQ ID NO.: 91, 3'→5' direction 1 to 903 of SEQ ID NO.: 90, nucleic acid sequence encoded (corresponding to 60321 to 61229 of SEQ ID No.: 51)(gene 33.5)

## Claims

1. A recombinant varicella-zoster virus.

2. A recombinant varicella-zoster virus of claim 1, comprising BAC vector sequence.

3. The recombinant varicella-zoster virus of claim 2, wherein at least part of the BAC vector sequence is inserted into a non-essential region of a varicella-zoster virus genome.

4. The recombinant varicella-zoster virus of claim 3, wherein the non-essential region is selected from the group consisting of the following regions:
the region in the ORF of gene 7, the region in the ORF of gene 8, the region in the ORF of gene 9, the region in the ORF of gene 10, the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, and the region flanking the ORF of gene 70.

5. The recombinant varicella-zoster virus of claim 4, wherein the non-essential region is the region flanking the ORF of gene 11, or the region flanking the ORF of gene 12.

6. The recombinant varicella-zoster virus of claim 2, wherein at least part of the BAC vector sequence is inserted into the region in the ORF of gene 62 of a varicella-zoster virus genome.

7. The recombinant varicella-zoster virus of claim 2, wherein the BAC vector sequence comprises recombinant protein dependent recombinant sequence.

8. The recombinant varicella-zoster virus of claim 2, wherein the BAC vector sequence comprises a selectable marker.

9. The recombinant varicella-zoster virus of claim 8, wherein the selectable marker is a drug selectable marker.

10. The recombinant varicella-zoster virus of claim 2, wherein the selectable marker is a gene encoding green fluorescent protein.

11. The recombinant varicella-zoster virus of claim 2, wherein the varicella-zoster virus genome is derived from a wild type strain.

12. The recombinant varicella-zoster virus of claim 2, wherein the varicella-zoster virus genome is derived from a mutant type strain.

13. The recombinant varicella-zoster virus of claim 2, wherein the varicella-zoster virus genome is derived from Oka vaccine strain.

14. The recombinant varicella-zoster virus of claim 2, wherein the varicella-zoster virus genome have mutations in gene 62 and gene 6.

15. The recombinant varicella-zoster virus of claim 14, wherein the gene 62 comprises at least the base substitutions of the following (a)-(d) in SEQ ID NO. 5:
(a) base substitution at position 2110 for G;
(b) base substitution at position 3100 for G;
(c) base substitution at position 3818 for C; and
(d) base substitution at position 4006 for G,
and, the gene 6 comprises at least a base substitution at position 5745 for G, in SEQ ID NO. 8.

16. The recombinant varicella-zoster virus of claim 2, wherein the BAC vector sequence comprises the sequence set forth in SEQ ID NO.: 7.

17. A pharmaceutical composition comprising the virus of claim 1.

18. The pharmaceutical composition of claim 17, wherein the composition is in the form of a vaccine.

19. A vector comprising a varicella-zoster virus essential gene and a BAC vector sequence other than the gene 62.

20. The vector of claim 19, further comprising the gene 62.

21. The vector of claim 19, wherein a mammalian cell produces a varicella-zoster virus when the vector is introduced into the mammalian cell.

22. The vector of claim 19, wherein a portion where a sequence derived from the varicella-zoster virus genome is linked to the BAC vector sequence is within a non-essential region of the varicella-zoster virus genome.

23. The vector of claim 22, wherein the non-essential region is selected from the group consisting of the following regions of:
the region in the ORF of gene 7, the region in the ORF of gene 8, the region in the ORF of gene 9, the region in the ORF of gene 10, the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.

24. The vector of claim 23, wherein the portion for linking is the region flanking the ORF of gene 11 and the region flanking the ORF of gene 12.

25. The vector of claim 19, wherein a portion where a sequence derived from the varicella-zoster virus genome is linked to the BAC vector sequence is in the ORF of gene 62 of the varicella-zoster virus genome.

26. The vector of claim 19, wherein the BAC vector sequence comprises recombinant protein dependent recombinant sequence.

27. The vector of claim 19, wherein the BAC vector sequence comprises a selectable marker.

28. The vector of claim 27, wherein the selectable marker is a drug selectable marker.

29. The vector of claim 27, wherein the selectable marker is a gene encoding green fluorescent protein.

30. The vector of claim 19, wherein the varicella-zoster virus genome is derived from a wild type strain.

31. The vector of claim 19, wherein the varicella-zoster virus genome is derived from a mutant type strain.

32. The vector of claim 19, wherein the varicella-zoster virus genome is derived from Oka vaccine strain.

33. The vector of claim 19, wherein the varicella-zoster virus genome have mutations in gene 62 and gene 6.

34. The vector of claim 33, wherein the gene 62 comprises at least the base substitutions of the following (a)-(d) in SEQ ID NO. 5:
(a) base substitution at position 2110 for G;
(b) base substitution at position 3100 for G;
(c) base substitution at position 3818 for C; and
(d) base substitution at position 4006 for G,
and, the gene 6 comprises at least a base substitution at position 5745 for G, in SEQ ID NO. 8.

35. The vector of claim 19, wherein the BAC vector sequence comprises the sequence set forth in SEQ ID NO.: 7.

36. A cell comprising the vector of claim 19.

37. The cell of claim 36, wherein the cell is a bacterial cell.

38. The bacterial cell of claim 37, wherein the bacterial cell is E. *coli.*

39. The cell of claim 36, wherein the cell is a mammalian cell.

40. The mammalian cell of claim 39, wherein the mammalian cell is derived from human.

41. A virus produced by the mammalian cell of claim 39.

42. A pharmaceutical composition comprising the virus of claim 41.

43. The pharmaceutical composition of claim 42, wherein the composition is in the form of a vaccine.

44. A method to produce recombinant varicella-zoster virus, comprising:
introducing a vector comprising a varicella-zoster virus genome essential gene other than gene 62 and BAC vector sequence into a mammalian host cell; and
culturing the mammalian host cell to produce recombinant varicella-zoster virus.

45. The method of claim 43, wherein the vector further comprises the gene 62.

46. The method of claim 44, wherein the mammalian host cell is derived from human.

47. The method of claim 44, wherein the BAC vector sequence comprises at least two recombinant protein dependent recombinant sequences.

48. The method of claim 47, further comprising a step of recombination between the two recombinant protein dependent recombinant sequences.

49. The method of claim 44, wherein a portion where a sequence derived from the varicella-zoster virus genome is linked to the BAC vector sequence is within a non-essential region of the varicella-zoster virus genome.

50. The method of claim 49, wherein the non-essential region is selected from the group consisting of the following regions of:
the region in the ORF of gene 7, the region in the ORF of gene 8, the region in the ORF of gene 9, the region in the ORF of gene 10, the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.

51. The vector of claim 50, wherein the portion which is linked is the region flanking the ORF of gene 11 or the region flanking the ORF of gene 12.

52. The vector of claim 44, wherein a portion where a sequence derived from the varicella-zoster virus genome is linked to the BAC vector sequence is in the ORF of gene 62 of the varicella-zoster virus genome.

53. The method of claim 44, wherein the BAC vector sequence comprises recombinant protein dependent recombinant sequence.

54. The method of claim 44, wherein the BAC vector sequence comprises a selectable marker.

55. The method of claim 54, wherein the selectable marker is a drug selectable marker.

56. The method of claim 54, wherein the selectable marker is a gene encoding green fluorescent protein.

57. The method of claim 44, wherein the varicella-zoster virus genome is derived from a wild type strain.

58. The method of claim 44, wherein the varicella-zoster virus genome is derived from a mutant type strain.

59. The method of claim 44, wherein the varicella-zoster virus genome is derived from Oka vaccine strain.

60. The method of claim 44, wherein the varicella-zoster virus genome has mutations in gene 62 and gene 6.

61. The method of claim 60, wherein the gene 62 comprises at least the base substitutions of the following (a)-(d) in SEQ ID NO. 5:
(a) base substitution at position 2110 for G;
(b) base substitution at position 3100 for G;
(c) base substitution at position 3818 for C; and
(d) base substitution at position 4006 for G,
and, the gene 6 comprises at least a base substitution at position 5745 for G, in SEQ ID NO. 8.

62. The method of claim 44, wherein the BAC vector sequence comprises the sequence set forth in SEQ ID NO.: 7.

63. A virus produced by the method of claim 44.

64. A pharmaceutical composition comprising the virus of claim 63.

65. The pharmaceutical composition of claim 64, wherein the composition is in the form of a vaccine.

66. A method to introduce a mutation into the vector of claim 19, comprising:
introducing the vector into a bacterial host cell;
introducing a plasmid vector comprising a fragment consisting of a portion of varicella-zoster virus genome into the bacterial host cell, wherein the fragment has at least one mutation;
culturing the bacterial host cell;
isolating a vector having BAC sequence from the cultured bacterial host cell.

67. A method to introduce a mutation into the vector of claim 19, comprising:
introducing the vector into a bacterial host cell;
introducing a first plasmid vector comprising a first fragment consisting of a portion of varicella-zoster virus genome into the bacterial host cell, wherein the first fragment has at least one mutation;
introducing a second plasmid vector comprising a second fragment consisting of a portion of varicella-zoster virus genome into the bacterial host cell, wherein the second fragment has at least one mutation, and the first fragment is different from the second fragment;
culturing the bacterial host cell;
isolating a vector having BAC sequence from the cultured bacterial host cell.

68. A nucleic acid cassette comprising a first fragment which can recombine with varicella-zoster virus genome in a bacterial cell, BAC vector sequence, and a second fragment which can recombine with varicella-zoster virus genome in a bacterial cell,
wherein the both ends of the BAC sequence are linked to the first and the second fragment, respectively.

69. The nucleic acid cassette of claim 68, wherein the first fragment and the second fragment are at least 1kb.

70. The nucleic acid cassette of claim 68, wherein the first fragment and the second fragment are at least 1.5kb.

71. The nucleic acid cassette of claim 68, wherein the first fragment and the second fragment are at least 2kb.

72. The nucleic acid cassette of claim 68, wherein the first fragment and the second fragment are at least 80% identical with a varicella-zoster virus genome sequence.

73. The nucleic acid cassette of claim 68, wherein the first fragment and the second fragment are at least 85% identical with a varicella-zoster virus genome sequence.

74. The nucleic acid cassette of claim 68, wherein the first fragment and the second fragment are at least 90% identical with a varicella-zoster virus genome sequence.

75. The nucleic acid cassette of claim 68, wherein the first fragment and the second fragment are at least 95% identical with a varicella-zoster virus genome sequence.

76. The nucleic acid cassette of claim 68, wherein each of the first fragment and the second fragment are independently selected from the group consisting of the following regions of varicella-zoster virus genome:
the region in the ORF of gene 7, the region in the ORF of gene 8, the region in the ORF of gene 9, the region in the ORF of gene 10, the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 62, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.

77. The nucleic acid cassette of claim 68, wherein each of the first fragment and the second fragment is independently at least 80% identical with the region selected from the group consisting of the following regions of varicella-zoster virus genome:
the region in the ORF of gene 7, the region in the ORF of gene 8, the region in the ORF of gene 9, the region in the ORF of gene 10, the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 62, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.

78. The nucleic acid cassette of claim 68, wherein each of the first fragment and the second fragment is independently at least 85% identical with the region selected from the group consisting of the following regions of varicella-zoster virus genome:
the region in the ORF of gene 7, the region in the ORF of gene 8, the region in the ORF of gene 9, the region in the ORF of gene 10, the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 62, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.

79. The nucleic acid cassette of claim 68, wherein each of the first fragment and the second fragment is independently at least 90% identical with the region selected from the group consisting of the following regions of varicella-zoster virus genome:
the region in the ORF of gene 7, the region in the ORF of gene 8, the region in the ORF of gene 9, the region in the ORF of gene 10, the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 62, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.

80. The nucleic acid cassette of claim 68, wherein each of the first fragment and the second fragment is independently at least 95% identical with the region selected from the group consisting of the following regions of varicella-zoster virus genome:
the region in the ORF of gene 7, the region in the ORF of gene 8, the region in the ORF of gene 9, the region in the ORF of gene 10, the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 62, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.

81. The nucleic acid cassette of claim 68, wherein the first fragment and the second fragment are derived from different regions.

82. The nucleic acid cassette of claim 72, wherein each of the first fragment and the second fragment are independently from the region flanking the ORF of gene 11 or the region flanking the ORF of gene 12.

83. The nucleic acid cassette of claim 68, wherein the BAC vector sequence comprises recombinant protein dependent recombinant sequence.

84. The nucleic acid cassette of claim 68, wherein the BAC vector sequence comprises a selectable marker.

85. The nucleic acid cassette of claim 84, wherein the selectable marker is a drug selectable marker.

86. The nucleic acid cassette of claim 68, wherein the selectable marker is a gene encoding green fluorescent protein.

87. The nucleic acid cassette of claim 68, wherein the varicella-zoster virus genome is derived from a wild type strain.

88. The nucleic acid cassette of claim 68, wherein the varicella-zoster virus genome is derived from a mutant type strain.

89. The nucleic acid cassette of claim 68, wherein the varicella-zoster virus genome is derived from Oka vaccine strain.

90. The nucleic acid cassette of claim 68, wherein the BAC vector sequence comprises the sequence set forth in SEQ ID NO.: 7.

91. The nucleic acid cassette of claim 68, having a nucleic acid sequence set forth in SEQ ID NO.: 2.
